# EUROPÄISCHE PATENTSCHRIFT

(11) **EP 2 809 803 B1**
(45) Veröffentlichungstag und Bekanntmachung des Hinweises auf die Patenterteilung: **03.10.2018**
(21) Anmeldenummer: 13702227.3
(22) Anmeldetag: 01.02.2013
(51) Int. Cl.: C12Q 1/68

(54) **GEMULTIPLEXTE DIGITAL PCR**
MULTIPLEXED DIGITAL PCR
PCR NUMÉRIQUE MULTIPLEXÉE

(30) Priorität: 01.02.2012 DE 102012100824
(43) Veröffentlichungstag der Anmeldung: 10.12.2014
(73) Patentinhaber: Albert-Ludwigs-Universität Freiburg, 79085 Freiburg (DE)
(72) Erfinder: HOFFMANN, Jochen, 71229 Leonberg (DE); ROTH, Günter, 79110 Freiburg (DE)
(74) Vertreter: Hertin und Partner Rechts- und Patentanwälte PartG mbB
(86) Internationale Anmeldenummer: PCT/EP2013/052063
(87) Internationale Veröffentlichungsnummer: WO 2013/113889

(56) Entgegenhaltungen:
- WO-A1-2010/077618
- WO-A1-2010/100265
- WO-A2-2008/022332
- WO-A2-2010/019388
- US-A1- 2005 220 675
- QI ZONGTAI ET AL: "Digital analysis of the expression levels of multiple colorectal cancer-related genes by multiplexed digital-PCR coupled with hydrogel bead-array.", THE ANALYST 7 JUN 2011, Bd. 136, Nr. 11, 7. Juni 2011 (2011-06-07) , Seiten 2252-2259, XP002692676, ISSN: 1364-5528
- BHAT SOMANATH ET AL: "Single molecule detection in nanofluidic digital array enables accurate measurement of DNA copy number", ANALYTICAL AND BIOANALYTICAL CHEMISTRY, SPRINGER, BERLIN, DE, Bd. 394, Nr. 2, 1. Mai 2009 (2009-05-01), Seiten 457-467, XP002632899, ISSN: 1618-2650, DOI: 10.1007/S00216-009-2729-5 [gefunden am 2009-03-15]
- COHEN DAWN E ET AL: "Self-digitization of sample volumes.", ANALYTICAL CHEMISTRY 1 JUL 2010, Bd. 82, Nr. 13, 1. Juli 2010 (2010-07-01), Seiten 5707-5717, XP002692677, ISSN: 1520-6882
- WANG JUN ET AL: "A novel nanofluidic digital-PCR platform to study MET receptor genomic amplification and mutation in lung cancer", AMERICAN ASSOCIATION FOR CANCER RESEARCH. PROCEEDINGS OF THE ANNUAL MEETING, AMERICAN ASSOCIATION FOR CANCER RESEARCH, US, Bd. 50, 1. April 2009 (2009-04-01), Seite 1254, XP009163130, ISSN: 0197-016X
- , 2 October 2011 (2011-10-02), XP055047304, Retrieved from the Internet: URL:http://www.rsc.org/images/LOC/2011/PDF s/Papers/302_0209.pdf [retrieved on 2012-12-10]

## Beschreibung

Die Erfindung betrifft ein Verfahren zum Nachweis von mindestens einem Zielmolekül und/oder Produktmolekül, wobei Reaktionskomponenten in definierten Bereichen einer Grundfläche und/oder Abdeckvorrichtung bereitgestellt werden, wobei es mindestens zwei definierte Bereiche mit unterschiedlichen Reaktionskomponenten gibt und wobei eine Amplifikationsreaktion durchgeführt wird. Die Erfindung betrifft außerdem eine Vorrichtung zur Durchführung des genannten Verfahrens.

### STAND DER TECHNIK

Im Stand der Technik sind zahlreiche molekularbiologische Untersuchungsmethoden beschrieben, die die Detektion und gegebenenfalls Analyse einer biologischen Probe ermöglichen. Eine dieser Methoden ist die Detektion und parallele Analyse von mehreren tausend Einzelnachweisen in einer geringen Menge biologischen Probenmaterials mittels eines Mikroarray. Es gibt verschiedene Formen von Mikroarrays, die manchmal auch als "Genchips" oder "Biochips" bezeichnet werden, weil sie wie ein Computerchip viele Informationen auf kleinstem Raum enthalten können.

Mikroarrays erlauben die hoch-parallele Detektion verschiedener Zielmoleküle auf einem typischerweise planaren Substrat. Die immobilisierten Sondenmoleküle eines Mikroarrays werden auf einem geeigneten Substrat im Laufe des Herstellungsprozesses an einer definierten Stelle (Spot) eines Rasters (Array) durch Übertragung geringer Flüssigkeitsvolumina immobilisiert beziehungsweise synthetisiert. Diese zweidimensionale, ortsaufgelöste Immobilisierung von Fängermolekülen kann so gestaltet sein, dass Nukleinsäuren oder Peptide beziehungsweise Proteine detektiert werden können. In der Regel erlauben in situ-Lithographieverfahren nur die Synthese kurzer Oligonukleotide beziehungsweise Aminosäureketten. Die hergestellten DNA-Mikroarrays können unter geeigneten Bedingungen über Monate, Protein-Arrays dagegen nur für eine kurze Zeit funktionell aufbewahrt werden.

Für eine DNA Mikroarray-Analyse wird die zu untersuchende Probe mit einem geeigneten Puffer vermischt und in entsprechender Weise mit dem Mikroarray inkubiert, so dass typischerweise bei komplementären Sequenzabschnitten ein Hybridisierungsereignis stattfindet. Aufgrund der geringen Sensitivität von Mikroarray-Systemen wird die Probe im Falle von nachzuweisender Nukleinsäure in einem vorgelagerten Reaktionsschritt amplifiziert (z. B. mittels Polymerase Chain Reaction (PCR), RT-PCR oder Whole Genome Amplification (WGA)) und für eine Detektion zum Beispiel mit einem Fluoreszenzfarbstoff markiert oder auf dem Mikroarray mit einer zusätzlichen Detektionssonde inkubiert. Peptide und Proteine können nicht enzymatisch amplifiziert werden und werden durch Aufreinigungen der zu untersuchenden Probe aufkonzentriert. Dem gegenüber stehen Ansätze, die eine Signalverstärkung auf dem Mikroarray nach erfolgter Hybridisierung, z.B. mittels Rolling Circle Amplification (RCA), durchführen. Diese Vorgehensweise umfasst mehrere zeitintensive Arbeitsschritte und erhöht die Gefahr für Ungenauigkeiten und Kontaminationen. Mikroarrays werden typischerweise bei Expressionsanalysen, sowie dem Nachweis von Pathogenen oder Resistenz-Markern verwendet. Ein Überblick über verschiedene Herstellungstechniken und Anwendungen ist für den Fachmann aus dem Stand der Technik bekannt.

Mikroarray-Analysen umfassen mehrere Arbeitsschritte, nämlich typischerweise die Auswahl der Sequenz eines immobilisierten Fängermoleküls, die Probenvorbereitung und die Amplifikation, die Hybridisierung beziehungsweise die Inkubation gefolgt von anschließenden Waschschritten sowie Signalmessung und Datenverarbeitung. Die im Stand der Technik bisher beschriebenen Mikroarrays basieren auf dem Prinzip der direkten Wechselwirkung zwischen Ziel- und immobilisierten Fängermolekül, daher muss ein geändertes Fängermolekül verwendet werden, sobald ein abweichendes Zielmolekül detektiert werden soll. Dies bedingt prinzipiell die Herstellung eines geänderten Arrays und ist ein wesentlicher Zeit- und Kostenfaktor. Aufgrund des aufwändigen Herstellungsprozesses, werden aus wirtschaftlichen Gründen größere Stückzahlen eines Sequenzlayouts gefertigt. Somit bietet das Arbeiten mit Mikroarrays geringe Flexibilität in Bezug auf das nachzuweisende Zielmolekül, da ein geändertes Sequenzlayout hohe Folgekosten und einen immensen Prozessierungsaufwand nach sich zieht.

Ferner sind im Stand der Technik universelle Mikroarrays offenbart. Universelle Nukleinsäure-Mikroarrays, bei denen die Sequenz der immobilisierten Fängermoleküle unabhängig von der Zielsequenz ist, sind kommerziell erhältlich, z. B. Affymetrix "GeneChip Universal Tag Arrays". Diese Verfahren basieren auf einem universellen Mikroarray-Sequenzlayout, bei dem die immobilisierten Oligonukleotide (ZIP code beziehungsweise Universal Tag) unabhängig von der nachzuweisenden Sequenz sind und nicht mit dieser interferieren. Bei einer typischen Nachweisreaktion wird die Zielsequenz in der Regel in einem gesonderten Reaktionsgefäß amplifiziert und gegebenenfalls aufgereinigt. Anschließend erfolgt ein Ligationsschritt, bei dem in Anwesenheit der Zielsequenz eine spezifische Detektionssonde und eine Fluoreszenzsonde direkt nebeneinander an der Basenabfolge der Zielsequenz hybridisieren. Die Detektionssonde besitzt einen Zielsequenzunspezifischen Überhang (complementary ZIP code, cZIP code), welcher als Adressierungssequenz dient und komplementär zu einer ZIP code-Sonde des Mikroarrays ist. Durch die Ligation entsteht ein Produkt aus Detektions- und Fluoreszenzsonde, welches für eine Hybridisierung auf einem ZIP code-Mikroarray verwendet wird. Ein Fluoreszenzsignal an einem spezifischen ZIP code-Spot ist ein indirekter Hinweis auf die Anwesenheit der Zielsequenz im Reaktionsmix. Als Festphase können auch kodierte Mikrokugeln (Beads) verwendet werden. Dabei wird jedem Fängermolekül ein eindeutig identifizierbares Bead zugewiesen, was durch eine definierte Nukleotidsequenz oder Färbung beziehungsweise Intensität erreicht wird. Durch diese Zuordnung kann in einem späteren Arbeitsschritt eine automatisierte Detektion des Beads sowie die parallele Analyse durchgeführt werden. Diese Methode besitzt den Vorteil, dass die Beads in der Flüssigphase homogenisiert vorliegen und die daraus resultierenden Reaktionskinetiken höher sind als bei vergleichbaren Flüssig-/Festphaseninteraktionen.

Weiterhin ist im Stand der Technik die PCR beschrieben. Die PCR ist eine vorzugsweise temperaturgesteuerte, enzymatische Reaktion, die aufgrund einer vorliegenden DNA (Probenfragment) ein Amplifikat desselben herstellen kann. Die Amplifikation erfolgt im Wesentlichen mithilfe von Enzymen, Energie und dNTPs (Desoxyribonukleosidtriphosphat). dNTPs sind molekulare Monomere, nämlich dATP (Desoxyadenosintriphosphat), dGTP (Desoxyguanosintriphosphat), dTTP (Desoxythymidintriphosphat), dCTP (Desoxycytidintriphosphat), die für die Synthese eines DNA-Stranges während einer Amplifikation benötigt werden. Falls als Probenfragment RNA zur Amplifikation vorliegt, wird anstelle einer DNA Polymerase eine RNA Polymerase und anstelle von dTTP wird dUTP (Desoxyuridintriphosphat) verwendet.

Eine Form der PCR ist die digitale PCR (dPCR). Eine Möglichkeit die Digitalität zu erreichen ist es, eine Probe so verdünnen, dass in einem Reaktionsvolumen (oder Reaktor oder Mikroreaktor) entweder kein (=0) oder ein (=1) DNA-Molekül vorliegt und somit eine hohe Zahl von PCR Reaktionen durchgeführt werden kann. Durch die enzymatische PCR-Amplifikation und Quantifizierung der Amplifikate kann festgestellt werden, ob das Reaktionsvolumen negativ (=̂ 0) oder positiv (=̂ 1) ist. In einem Reaktionsraum wird nur dann ein Amplifikat generiert, wenn in dem Reaktionsraum mindestens ein einziges DNA Molekül vorlag. Durch eine Auszählung der positiven Signale kann die zu amplifizierende Probe quantifiziert werden. Darüber hinaus kann das Amplifikat auch an eine Festphase gebunden werden (z.B. über eine Primer Verlängerungsreaktion), wenn die dPCR mit einer Festphasen PCR verbunden ist.

In der Literatur sind prinzipiell zwei unterschiedliche Systeme zur Durchführung der digitalen PCR beschrieben: Das erste System umfasst die sogenannte Öl-Emulsions-PCR (emPCR), die ein tröpfchenbasiertes System darstellt. Hierbei bilden Millionen von Tröpfchen, die einzelne DNA-Moleküle enthalten, PCR-Reaktionsräume, wobei die Tröpfchen mikrofluidisch in einer Ölmatrix generiert werden. Nach der PCR-Reaktion können die Zielmoleküle durch Quantifizierung der positiven Signale in den Tröpfchen bestimmt werden. Um einen emPCR-Ansatz zum Nachweis von mehr als einem Genomabschnitt zu verwenden, also die emPCR multiplexfähig zu machen, wird die amplifizierte DNA entweder sequenziert, was teuer und zeitaufwändig ist, oder die Sequenzen werden mit Hilfe einem Mix aus Taqmansonden unterschieden. Diese Methode ist jedoch zurzeit auf weniger als fünf Zielmoleküle limitiert. Obwohl die ölbasierten Amplifikationsmethoden eine hohe Anzahl an möglichen Reaktionen erzielen, leiden die emPCR Systeme darunter, dass sie nur eine unzureichende Amplifikationseffizienz und eine komplizierte Durchführung aufweisen. So sind zahlreiche und vor allem aufwendige Arbeitsschritte sowie teure Geräte notwendig. Ein weiterer intrinsischer Nachteil der emPCR ist, dass die Herstellung der Tröpfchen sehr zeitaufwändig ist und die Tröpfchen fusionieren können, was wiederum zu falschen Ergebnissen führen kann.

Das zweite System im Stand der Technik zur Durchführung einer digitalen PCR umfasst sogenannte PCR-Mikroreaktoren, die auf einem Chip realisiert sind und durch eine geschlossene Architektur gekennzeichnet sind. Die Mikroreaktoren sind entweder mechanisch oder durch eine zweite Flüssigphase (z. B. Öl) verschlossen. Im Gegensatz dazu verwenden offene Systeme Mikroreaktoren, die durch eine Membran, Silikonschichten, Mineralöl oder durch einen SlipChip-System verschlossen werden. Ein wesentlicher Nachteil der on-Chip digitalen PCR-Systeme ist, dass in den Systemen jeweils nur eine Zielsequenz pro Chip und Probe analysiert werden kann.

Nachteilig bei den im Stand der Technik offenbarten Systemen ist weiterhin, dass diese bedingt durch ihre Strukturen eine geringe Möglichkeit zum Multiplexen ermöglichen und zudem von speziellen Laborgeräten abhängig sind. Außerdem nachteilig ist, dass im Stand der Technik meist Spezialgeräte für die Prozessierung benötigt werden. Ein Hauptnachteil ist, dass Detektion nur weniger Zielsequenzen ("Targets") möglich ist. Je nach System ist die Zahl der detektierbaren Zielsequenzen auf ein oder maximal 144 Targets (Applied Bioscience) beschränkt.

Weiterhin sind die Systeme aus dem Stand der Technik unflexibel und komplex in dem Sinne, dass die Abänderung des Systems auf andere Zielsequenzen einen größeren Aufwand nach sich zieht. Derzeit ist es noch notwendig eine Probenlösung vor der digitalen PCR zu quantifizieren, um verwertbare Ergebnisse zu erhalten.

Bei den meisten Systemen aus dem Stand der Technik haben die Reaktoren beziehungsweise die Reaktionsräume eine einheitliche Größe, weshalb bei Proben zunächst die Konzentration bestimmt werden muss, um genau in das Reaktionsregime von weniger als einer DNA Sequenz pro Kavität zu gelangen. Eine zu hoch konzentrierte DNA erzeugt nur positive Kavitäten und damit eine Sättigung, eine zu niedrig konzentrierte DNA gibt nur wenige Einzelsignale und damit eine sehr schlechte Statistik.

Im Stand der Technik werden außerdem in der US 6,482,593 eine Methode und Vorrichtung für die Detektion von DNA-Molekülen mit Hilfe eines Sensorarrays offenbart. Dieses Array ist ein strukturiertes Multiwell- Array, das an diskreten Stellen immobilisierte Biosensoren aufweist. Eine Amplifikationsreaktion findet jedoch nicht statt.

In der Druckschrift WO 2010/077618 wird ein Array beschrieben, welcher sich durch eine Vielzahl von Reaktionskammern auszeichnet. Es handelt sich um einen Chip mit einer sehr komplexen Geometrie aus Steuer- und Befüllkanälen, der zudem über Ventile verfügt und durch Schließen und Öffnen dieser ein Flusssystem schafft, mit dem die Proben in Reaktionskammern geladen werden können.

In der WO 2010/019388 A2 wird ein mikrofluidischer Chip mit einem digitalen Array und Chemikalien in Reaktionskammern offenbart. Die Geometrie umfasst einen Kanal mit seitlichen Ausbuchtungen, welche über eine Zuleitung mit einem Reaktionsmix befüllt werden und über eine Steuerleitung kontrolliert werden können. Eine räumliche Abtrennung der einzelnen Reaktionskomponenten wird über eine Phasentrennung mittels Öl induziert. Es handelt sich um einen geschlossenen Chip, dessen einzelne Zuleitungen separat befüllt werden müssen.

In der Druckschrift von Qi Zongtai et al ("Digital analysis oft he expression levels of multiple colorectal cancer-related genes by multiplexed digital-PCR coupled with hydrogel beadarray", The Analyst, Bd. 136, Nr. 11, June 2011, S. 2252-2259) wird eine räumliche und zeitliche Trennung zwischen PCR und Nachweis der Produkte bzw. deren Identifikation offenbart. Hierbei wird eine räumliche Trennung nur zur Fixierung von Beads genutzt.

In der Druckschrift von Bath Somanath et al ("Single molecule detection in nanofluidic digital array enables accurate measurement of DNA copy number", Analytic and bioanalytical chemistry, Bd. 394, Nr.2, Mai 2009, S. 457-467) wird ein Chip gemäß der WO 2010/077618 Kombination mit einer digitalen PCR offenbart.

In der WO 2010/100265 A1 wird eine Methode des Array-Kopierens beschrieben. Ein primärer Array wird als Vorlage zur Herstellung von Replikaten verwendet. Dabei wird die DNA in Kavitäten amplifiziert und an eine Abdeckung über Adaptoren gebunden.

Im Stand der Technik ist außerdem die WO 2008/022332 A2 bekannt. Hier wird die Herstellung von Produktmolekülen durch eine biologische Reaktion auf einer Oberfläche mit Ausgangsmolekülen sowie die Übertragung dieser Produkte auf eine zweite Oberfläche unter Beibehaltung der räumlichen Anordnung beschrieben. Eine Vervielfältigung der Produktmoleküle findet nicht statt. Vielmehr wird cDNA hergestellt und an die Abdeckung gebunden.

In der Veröffentlichung von Hoffmann et al ("Single-Molecule PCR in a picowell array simultaniously immobilizing PCR products to a PDMS coverslide") wird ein Mikroarray mit Amplifikationsreagenzien befüllt und mit einer Abdeckung verschlossen, wobei die Abdeckung mit immobilisierten Rückwärtsprimern beschichtet ist. Das PCR Produkt wird am Deckel fixiert, wobei eine grobe Ortskodierung ermöglicht wird.

Für eine Real-time Detektion der Reaktion sind immer komplexe und teure Geräte nötig, was zusätzlich nachteilig ist. Die Komponenten aktueller Systeme sind teilweise komplex und dadurch teuer in der Herstellung. Oftmals werden keine Einwegartikel angeboten.

Die Systeme im Stand der Technik die Öl für die Herstellung einzelner Reaktionskompartimente verwenden zeigen den Hauptnachteil der Instabilität der so geformter Reaktionskompartimente, da einzelne Tröpfchen während der Reaktion miteinander verschmelzen können und so die Aussagekraft der gesamten Reaktion verloren geht. Um mehrere Zielsequenzen identifizieren zu können, wird in vielen Systemen aus dem Stand der Technik sequenziert. Dies ist wiederum teuer und sehr zeitaufwendig. Weiterhin weisen viele Systeme eine schlechte Reaktionseffizienz auf oder benötigen eine komplizierte Reaktionsführung.

Es war demgemäß die Aufgabe der vorliegenden Erfindung, ein Verfahren und eine Vorrichtung bereitzustellen, welche nicht die Nachteile oder Mängel des Standes der Technik aufweisen.

### BESCHREIBUNG DER ERFINDUNG

Gelöst wird die Aufgabe durch die unabhängigen Ansprüche. Vorteilhafte Ausführungsformen ergeben sich aus den Unteransprüchen.
Die Erfindung betrifft in einer ersten bevorzugten Ausführungsform ein Verfahren zum Nachweis von mindestens einem Zielmolekül und/oder Produktmolekül, umfassend die folgenden Schritte
a) Bereitstellen einer Grundfläche mit offenen Kavitäten und einer Abdeckvorrichtung, wobei
   (i) auf der Grundfläche und/oder der Abdeckvorrichtung Reaktionskomponenten in definierten Bereichen vorhanden sind, wobei die definierten Bereiche auf der Grundfläche im Bereich der Kavitäten liegen und es mindestens zwei definierte Bereiche mit unterschiedlichen Reaktionskomponenten gibt, und
   (ii) die Grundfläche eine Flüssigkeits-Barriere aufweist, welche die Kavitäten der Grundfläche umrandet, und einen Bereich zur Bereitstellung einer Reaktionslösung aufweist, und
   (iii) die Abdeckvorrichtung Spots umfasst und ein Spot der Abdeckvorrichtung mehrere Kavitäten der Grundfläche abdeckt,
b) Zugabe der Reaktionslösung auf den Bereich zur Bereitstellung der Reaktionslösung, wobei die Reaktionslösung die Zielmoleküle enthält,
c) Auflegen der Abdeckvorrichtung auf die Grundfläche, so dass die Abdeckvorrichtung innerhalb der Flüssigkeits-Barriere angeordnet wird, wobei durch das Auflegen der Abdeckvorrichtung die Reaktionslösung auf die Kavitäten verteilt wird, die Reaktionslösung in die Kavitäten eindringt und nach dem Auflegen der Abdeckvorrichtung jede Kavität mit der Abdeckvorrichtung einen abgeschlossenen Reaktionsraum bildet,
d) Durchführen einer Amplifikationsreaktion, einer Nachweisreaktion und/oder einer Derivatisierungsreaktion, wobei die Reaktionslösung mit den Reaktionskomponenten in Kontakt kommt und Produktmoleküle entstehen, und
e) Nachweis von mindestens einem Zielmolekül und/oder Produktmolekül, wobei mindestens zwei unterschiedliche Abschnitte des Zielmoleküls und/oder Produktmoleküls in separaten abgeschlossenen Reaktionsräumen nachgewiesen werden und/oder mindestens zwei unterschiedliche Zielmoleküle und/oder Produktmoleküle in separaten abgeschlossenen Reaktionsräumen nachgewiesen werden.

Sobald die Abdeckvorrichtung und die Grundfläche zueinander angeordnet werden, formen sich eine Vielzahl von Reaktionsräumen. Die Reaktionsräume werden durch die Kavitäten mitgebildet. Die Reaktionsräume sind dabei voneinander getrennt sodass keine Vermischung stattfinden kann. Es ist bevorzugt, dass die entstehenden Reaktionsräume abgeschlossen sind und jede Kavität einen Reaktionsraum bildet.

Es ist besonders bevorzugt, dass die Abdeckvorrichtung ein Mikroarray ist und die definierten Bereiche Spots des Mikroarrays sind. Ein Mikroarray im Sinne der Erfindung zeichnet sich bevorzugt durch die regelmäßige Anordnung von Spots auf einem Substratmaterial aus. Es ist bevorzugt, dass die Spots einen Durchmesser von 0,1 µm² - 10 cm² aufweisen. Die Geometrie der Spots kann bevorzugt quadratisch, rechteckig, rund, oval, dreieckig, hexagonal, oktagonal oder polygonal sein. Aber auch jede andere, hier nicht explizit genannte, Form kann unter bestimmten Vorraussetzungen wünschenswert und vorteilhaft sein. Es ist auch möglich, dass sich die Spots Einkerbungen in der Abdeckvorrichtung darstellen, wobei die Spots sich in ihrer Tiefe oder anderen Merkmalen unterscheiden können. Die Spots können außerdem in unterschiedlichen Anordnungen zueinander, regelmäßig oder unregelmäßig, angeordnet sein. Wenn als Abdeckvorrichtung ein Mikroarray verwendet wird, ist es bevorzugt, dass die Reaktionskomponenten in dem Mikroarray, besonders bevorzugt in den Spots des Mikroarrays bereitgestellt, ganz besonders bevorzugt vorgelagert werden.

Die Reaktionskomponenten können hierbei unterschiedliche Bestandteile und Funktionen besitzen, wodurch in einer Vorrichtung (Grundfläche oder Abdeckvorrichtung) z. B. unterschiedliche Bereiche eines Zielmoleküls mittels einer PCR analysiert werden können. Auf einer Vorrichtung können demnach unterschiedliche Reaktionen durchgeführt werden, was den Arbeitsaufwand und die Kosten erheblich reduziert und außerdem ein praktisches Werkzeug z.B. für die Diagnose liefert.

Es ist außerdem bevorzugt, dass die definierten Bereiche in den Kavitäten der Grundfläche liegen. Die Geometrie der Kavitäten der Grundfläche kann bevorzugt quadratisch, rechteckig, rund, oval, dreieckig, hexagonal, oktagonal oder polygonal sein. Aber auch jede andere, hier nicht explizit genannte, Form kann unter bestimmten Vorraussetzungen wünschenswert und vorteilhaft sein. Es ist auch möglich, dass sich die Kavitäten in ihrer Tiefe oder anderen Merkmalen unterscheiden. Die Kavitäten können außerdem in unterschiedlichen Anordnungen zueinander, regelmäßig oder unregelmäßig, angeordnet sein. Die Grundfläche im Sinne der Erfindung kann auch als Chip bezeichnet werden, wobei die Kavitäten auch als Reaktoren bezeichnet werden können. Bei den Kavitäten im Sinne der Erfindung handelt es sich bevorzugt um Einzelkompartimente. In jeder Kavität kann somit bei Vorhandensein eines Zielmoleküls eine eigene Nachweisreaktion, z.B. eine PCR ablaufen.

Weiterhin bevorzug ist, dass die Grundfläche 1 bis 10^10, bevorzugt 10^2 bis 10^8, besonders bevorzugt 10^4 bis 10^6 Kavitäten aufweist und/oder die Kavitäten ein Volumen von 1 fL bis 1 mL besitzen.

Es ist außerdem vorteilhaft, dass die Grundfläche eine Flüssigkeits-Barriere aufweist, welche die Kavitäten der Grundfläche umrandet. Die Flüssigkeits-Barriere bezeichnet im Sinne der Erfindung insbesondere eine Barriere für die eingebrachte Reaktionslösung, so dass diese nicht über den Rand der Grundfläche hinaustritt. Die Barriere kann vorteilhafterweise eine physikalische und/oder chemische Barriere sein. Hierbei kann es sich bevorzugt um einen hydrophoben Bereich oder einen Rahmen handeln, der die Grundfläche umläuft und so eine Abgrenzung zur Umgebung darstellt. Auf beziehungsweise in den Innenbereich der Barriere kann die Abdeckvorrichtung aufgelegt werden, um abgeschlossene Reaktionsräume innerhalb der Vorrichtung herzustellen. Das bedeutet die Abdeckvorrichtung liegt vorzugsweise mindestens auf einem Anteil der Kavitäten auf. Dabei können mittels der Abdeckvorrichtung sowohl ein Teil oder auch alle Kavitäten abgedeckt werden. Die Barriere dient dazu, dass die Reaktionslösung ausschließlich in die Kavitäten gelangt. Im Sinne der Erfindung kann die Barriere insbesondere auch als Führungsschiene für Flüssigkeiten bezeichnet werden, da die Reaktionslösung so in die vorgesehenen Bereiche eindringen kann.

Es ist bevorzugt, dass nach Befüllung der Kavitäten und Verschluss mit der Abdeckvorrichtung, die Barriere die Abdeckvorrichtung umfasst und diese somit von einem erhabenen Rahmen umrandet wird. Durch diese Ausführungsform wird eine besonders gut abgeschlossene Vorrichtung zur Verfügung gestellt. Es ist außerdem bevorzugt, dass jede Kavität mit der Abdeckvorrichtung einen abgeschlossen Reaktionsraum bildet.

Es ist bevorzugt, dass die Reaktionskomponenten auf der Abdeckvorrichtung und/oder der Grundfläche flächig oder bereichsweise vorhanden sind. Bei der bereichsweisen Bereitstellung werden bevorzugt pro Spot oder pro Kavität ein, zwei oder mehrere unterschiedliche Reaktionskomponenten vorgelagert.

Die Reaktionskomponenten umfassen bevorzugt Primer, Sonden, interkalierende Farbstoffe, Polymerase, weitere Enzyme und/oder reaktionsverbessernde Komponenten. Es ist dabei besonders bevorzugt, dass die Reaktionskomponenten in den Kavitäten der Grundfläche und/oder den Sports der Abdeckvorrichtung vorgelagert sind. Diese Reaktionskomponenten können in einem Rasterformat mit einem Spottingverfahren (z.B. Topspotsystem) in räumlich begrenzte Bereiche der Grundfläche und/oder der Abdeckvorrichtung eingebracht werden.

Reaktionsverbessernde Komponenten umfassen vorzugsweise "bovine serum albumin" (BSA), Agarose, Tween, ultrageschallte Nukleinsäuren und/oder ein UNG-Verdausystem.

Die Länge der Primer liegt üblicher Weise zwischen 1 und 10^3 Einzelmolekülen. Primer im Sinne der Erfindung enthalten bevorzugt eine Anbindestelle für Zielmoleküle, bevorzugt DNA-Zielmoleküle. Die Primer können für die orientierte Anbindung an einer Oberfläche an einem oder mehreren Enden eine chemische Kopplungsgruppe enthalten. Diese Gruppe kann unter anderem Biotin, double-Biotin, NH2, SH, Acrydite oder eine Polynukleotidsequenz sein. Es kann sich außerdem um übliche Tags und Binder wie Streptavidin, His-Tag, Nickel-NTA, myc- oder flag-Tag oder ähnliches handeln. Die Primer können neben der nachweisspezifischen Sequenz zusätzlich noch Markierungssequenzen enthalten. Diese Markierungssequenzen können sowohl DNA, Fluorophore als auch andere Moleküle sein.

Die Primer können Flüssigphasenprimer oder Festphasenprimer sein. Wenn es sich um Festphasenprimer handelt, können diese entweder direkt oder indirekt immobilisiert sein. Verfahren für die direkte Immobilisierung von Nukleinsäuren können bevorzugt sein: Hydrogele, UV-Crosslinking, Kopolymerisation in Acrylamidgele, Affinitätsbindungen (avidinstreptavidin), ionische oder absorptive Bindungen. Verfahren für indirekte Immobilisierung verwenden Linkermoleküle, zum Beispiel homobifunktionale oder heterobifunktionale Linkermoleküle. Der homobifunktionale Linker kann beispielsweise PDITC sein. Generell ist die resultierende Anbindung der Primer an eine Festphase kompatibel zu Amplifikationsreaktionen und der eventuell nachfolgenden Derivatisierungen.

Weiterhin kann ein Spacermolekül zwischen der Kopplungsgruppe und Nukleinsäuresequenz vorhanden sein. Der Spacer kann unter anderem aus einer Kohlenstoffkette oder einer Polynukleotidsequenz (Poly-T Sequenz) bestehen.

Die Primer können zudem Promotorstellen und/oder Bindestellen für eine RNA-Polymerase oder ein zellfreies Expressionssystem enthalten.

Weiterhin kann nur einer der Primer an die Oberfläche gebunden sein und der andere löslich vorliegen (übliche Festphasen-PCR) oder beide Primer an die Oberfläche gebunden sein (entspricht einer Bridge-Amplifikation). Diese Verfahren unterscheiden sich jedoch vom Stand der Technik dahingehend, dass diese räumlich begrenzt sind. Die Bridgeamplifikation kann nur im Innenbereich einer Kavität stattfinden und die Kavität aufgrund der räumlichen Abtrennung nicht verlassen, obwohl die komplette Oberfläche um die Kavität eventuell auch eine Bridgeamplifikation zuließe.

Bevorzugter Weise werden die Reaktionskomponenten oder Teile davon an der Grundfläche und/oder der Abdeckfläche immobilisiert. Besonders bevorzugt ist, dass Reaktionskomponenten oder Teile davon kovalent an der Grundfläche und/oder der Abdeckvorrichtung gebunden sind. Wenn Primer an die Grundfläche und/oder Abdeckvorrichtung immobilisiert werden, wird zusätzlich die Durchführung einer Bridge Amplifikation ermöglicht. Diese Reaktion läuft dann ausschließlich über Festphasenprimer ab und es sind bevorzugt keine Flüssigphasenprimer im System vorhanden.

Das Verfahren kann daher bevorzugt auch eine Festphasen PCR (auch Solid-phase PCR oder SP-PCR) sein. Dabei handelt es sich um eine PCR Reaktion bei der einer der beiden Primer an einer Festphasen (z.B. der Abdeckvorrichtung immobilisiert ist). Über diesen Festphasenprimer wird das im Rahmen einer PCR generierte PCR Produkt an die Festphase gebunden.

Die Grundfläche im Sinne der Erfindung kann auch als Reaktorchip bezeichnet werden. Bei einem solchen Reaktorchip handelt es sich bevorzugt um einen "convenient-product", welches vorzugsweise als Einwegartikel angeboten wird.

Es ist außerdem vorteilhaft, dass ein Spot der Abdeckvorrichtung nach dem zueinander Anordnen von Abdeckvorrichtung und Grundfläche mehrere Kavitäten der Grundfläche abdeckt. Somit können die Produktmoleküle und/oder Zielmoleküle mehrerer Kavitäten auf einem Spot analysiert werden.

Es ist besonders bevorzugt, dass das Verfahren ein digitales Verfahren ist. Im Sinne der Erfindung bedeutet Digitalität bevorzugt, dass auf einen definierten Bereich im Schnitt ein oder kein Zielmolekül kommt. Digitalität im Sinne der Erfindung bedeutet aber auch, dass bei dem Nachweis von unterschiedlichen Zielmolekülen im Schnitt in einen definierten Bereich nur eine Art von Zielmolekül vorliegt, sodass es also pro Art von Zielmolekül mehr definierte Bereiche als Zielmoleküle gibt. Digitalität im Sinne der Erfindung bedeutet weiterhin bevorzugt auch, dass wenn mehrere Abschnitte eines Zielmoleküls nachgewiesen werden sollen im Schnitt in einem definierten Bereich nur ein Abschnitt eines Zielmoleküls nachgewiesen wird. In diesem Fall ist also die Zahl der Zielmoleküle nicht zwingend geringer als die Zahl der definierten Bereiche aber die vorgelagerten Reaktionskomponenten führen zur Digitalität, da nicht in jedem definierten Bereich die gleichen Reaktionskomponenten vorgelagert sind.

Die Funktionsweise des digitalen Verfahrens kann dabei unter anderem über die Geometrie der Kavitäten und/oder Spots oder auch über die Anzahl der Kavitäten und/oder Spots beeinflusst werden.

Die Erfindung vereint die Technologie von DNA Mikroarrays mit digitalen Reaktionen, bevorzugt digitalen PCR-Reaktionen, um so auf jedem Spot der Abdeckvorrichtung oder in jeder Kavität der Grundfläche eine digitale PCR durchführen zu können. Dies erlaubt den Nachweis von mehr als einem Zielmolekül oder mehreren Abschnitten eines Zielmoleküls. Durch dieses Multiplexen entsprechend eines Mikroarrays unter Beibehaltung der Vorteile der digitalen PCR kann das Verfahren im Sinne der Erfindung besonders bevorzugt in der Diagnostik eingesetzt werden. Multiplexing bezeichnet im Sinne der Erfindung insbesondere die Analyse zahlreicher einzelner Reaktionen zu einem Ergebnis.

Es ist besonders bevorzugt, dass der Amplifikationsschritt eine digitale PCR und/oder eine digitale Festphasen-PCR ist.

Es ist besonders bevorzugt, dass das Verfahren zur Durchführung einer digitalen PCR (dPCR) verwendet wird. Die Grundfläche wird dabei mit einer Probe, die im Sinne der Erfindung auch in einer Reaktionslösung enthalten sein kann, befüllt und mit der Abdeckvorrichtung verschlossen. Anschließend wird eine digitale PCR durchgeführt. Nach Benutzung kann die Grundfläche entsorgt werden.

Mit digitaler PCR im Sinne der Erfindung sind Reaktionen gemeint, bei denen die Digitalität auf unterschiedliche Weise erreicht werden kann. Zum einen ist es möglich, dass die Anzahl der definierten Bereicht (Kavitäten oder Spots) größer ist als die Anzahl der Zielmoleküle die sich auf die definierten Bereiche verteilen. Wenn unterschiedliche Zielmoleküle nachgewiesen werden, kann die Digitalität auch dadurch entstehen, dass die Zahl der definierten Bereiche (Kavitäten oder Spots) größer ist als jeweils eine Art der Zielmoleküle. Es ist aber auch bevorzugt, dass die Reaktionskomponenten vorgelagert werden und beispielsweise jeweils eine Art von Primern (zum Beispiel für ein bestimmtes Zielmolekül oder einen bestimmten Abschnitt eines Zielmoleküls) in geringerer Anzahl vorliegt als es definierte Bereiche (Kavitäten oder Spots) gibt.

Es war völlig überraschend, dass durch die Kombination von etablierten DNA Mikroarrays mit einer Grundfläche die Kavitäten für die digitale PCR enthält ein so omnipotentes und flexibles Detektionssystem geschaffen werden kann. Bevorzugt werden technisch gesehen die einmaligen Möglichkeiten der digitalen PCR mit den multiplexing Eigenschaften von Mikroarrays kombiniert. Durch besagte Kombination erhält dieses System herausragende Merkmale, welche die aus dem Stand der Technik bekannten Systeme weit übertreffen.

Es war sehr überraschend, dass auch Reaktionslösungen mit einer hohen Konzentration an Zielmolekülen keine Limitierung für die Vorrichtung und das Verfahren der Erfindung bedeuten und ebenfalls eine digitale PCR durchführbar ist. Auch für den Fall, dass die Zielmoleküle, bevorzugt DNA-Zielmoleküle, stark verdünnt vorliegen, kann überraschenderweise eine digitale PCR ausgeführt werden, indem ein großes Volumen analysiert wird. Mittels einer statistischen Methode (insbesondere der MPN/Most Probable Number -Methode - "Wahrscheinlichsten Anzahl") ist es zudem möglich, aus einer Vorrichtung mit einem großen Volumen auswertbare Daten zu extrahieren. Diese Methode wird vorzugsweise dann angewandt, wenn das Ergebnis zwar 0 und 1, aber auch "mehr" DNA umfasst. Die Methode ermittelt hierfür die positiven Ergebnisse einer Verdünnungsreihe und berechnet hieraus die wahrscheinlichste Anzahl der initialen DNA-Konzentration pro Volumen. Hierdurch kann die statistische Validität extrem verbessert werden. In einer bevorzugten Ausführungsform ist das digitale Verfahren eine Untergruppe der MPN-Methode. Dadurch, dass die Volumina der Kavitäten einfach an die Menge der DNA in einer zu analysierenden Probe angepasst werden können, ist die Analyse jeder erdenklichen DNA-Probe oder -Konzentration möglich. Somit kann ein wesentlicher Nachteil der im Stand der Technik beschriebenen Verfahren ausgeräumt werden, nämlich dass jegliche PCR-Methode durch die DNA-Konzentration limitiert ist. Dieser Nachteil kann vorzugsweise durch die Verdünnungsschritte in den Kavitäten umgangen werden.

Es kann auch bevorzugt sein, dass die Reaktionskomponenten in den Kavitäten der Grundfläche vorgelagert sind, sodass die Grundfläche einen Mirkoarray darstellt. In diesem Fall ist es auch möglich, dass auf der Abdeckvorrichtung Reaktionskomponenten (z.B. Primer und/oder Sonden) flächig aufgetragen wurden. in der

Der Erfindung stellt daher bevorzugt eine Kombinationserfindung dar. Denn bevorzugt werden die bekannten Elemente der PCR und des Mikroarrays miteinander kombiniert. Die überraschenden Effekte die hierdurch entstehen, sind zum einen die Genauigkeit der durchgeführten Verfahren und das hochgradige Multiplexen was durch die Erfindung erstmals auf einfache und kostengünstige Weise ermöglicht wird.

Der Nachweis beziehungsweise die Detektion der Zielmoleküle im Sinne der Erfindung kann auf unterschiedliche Weise erfolgen. Dem Fachmann sind z.B. unterschiedliche Verfahren bekannt, Amplifikate einer PCR Reaktion zu detektieren. Zum einen kann die Reaktion auf der Oberfläche der Abdeckvorrichtung oder der Grundfläche ablaufen. Es ist aber auch möglich, dass die Reaktion in dem Volumen, welches durch die Verdeckelung zwischen Abdeckvorrichtung und Grundfläche gebildeten wird, stattfinden. Es ist aber auch eine Kombination aller drei Mechanismen miteinander möglich.

Für die Detektion auf der Oberfläche der Abdeckvorrichtung oder der Grundfläche können zum Beispiel oberflächen-gebundene PCR Produkte mit interkalierenden Farbstoffen nachgewiesen werden. Es ist auch möglich den Nachweis über die Ankopplung von Streptavidin konjugierten Fluorophoren oder eine Hybridisierungsreaktion zu führen. Weiterhin kann in der digitalen PCR ein Signal auf einer Oberfläche erzeugt werden. Dies kann zum Beispiel durch eine RCA (Rolling Circle Amplification), die Hybridisierung eines fluoreszenten PCR Produkts an komplementäre Festphasenprimer, oder eine speziellen Detektionsassay geschehen. Sämtliche Methoden können als Endpunktmessung nach der Amplifikationsreaktion erfolgen. Mittels oberflächensensitiver Methoden z.B. konfokaler Laser-Mikroskopie, ATR-Messungen, Biacore etc. kann aber auch schon während der Reaktion eine real-time Messung erfolgen. Dies hat für bestimmte Verfahren den Vorteil, dass die Reaktion noch beeinflusst werden kann. Der Fachmann weiß, auf welche Art er einen Nachweis von Reaktionsprodukten führen kann und ist in der Lage eine geeignete Methode je nach Zielmolekül oder Produktmolekül auszuwählen ohne dabei selbst erfinderisch tätig zu werden.

Zum Beispiel kann eine Detektion direkt nach der Amplifikationsreaktion ohne spezifische Nachweisreaktion mittels interkalierender Farbstoffe (zum Beispiel Sybr Green oder Ethidium-Bromid) oder fluorophor gelabelten Primern in der Amplifikationsreaktion erfolgen. Hierzu sind dann ein Waschschritt und die Demontage nötig, damit die Grundfläche und/oder die Abdeckvorrichtung gewaschen werden kann.

Findet die Detektion nicht auf einer Oberfläche statt, kann diese während der Amplifikationsreaktion mittels eines interkalierenden Farbstoffes oder mittels eines passenden Sondensystems in real-time erfolgen. Hierfür sind insbesondere alle Methoden der herkömmlichen real-time PCR möglich wie direkt interkalierende Farbstoffe (Ethidiumbromid, Sybr Green), Taqman oder High-Probe Sonden etc. So kann während der Reaktion bereits der Anstieg der Fluoreszenz vermessen werden. Ein generiertes Amplifikat kann aber auch als Endpunkt vermessen werden. Die zuvor genannten Methoden sind hier ebenfalls verwendbar. In beiden Fällen kann in den jeweiligen Kavitäten oder dem Spot ein Fluoreszenzsignal detektiert werden.

Aufgrund des farblichen Multiplexens wie es bei der Taqman-PCR möglich ist, ist es hier ebenfalls möglich innerhalb der definierten Bereiche parallel bis zu 10 unterschiedliche Zielmoleküle oder Zielmolekülabschnitte zu amplifizieren. Somit ist es dann möglich in verschiedenen Farben eine digitale Amplifikationsreaktion durchzuführen und damit die Belegungsdichte des Systems entsprechend der Anzahl der verwendeten Farben zu erhöhen. Eine der Farben kann zudem als Referenz verwendet werden, die Auskunft gibt, ob eine Amplifikationsreaktion erfolgt ist und wie stark das Amplifikationssignal ist. Somit sind eine interne Kalibrierung und gleichzeitig eine Positiv-Kontrolle möglich, die es erlaubt auch schwache Signale als solche zu erkennen und starke Signale als Artefakte oder echte Messwerte zu bewerten.

Die Reaktionslösung enthält die Zielmoleküle. Außerdem bevorzugt ist, dass die Reaktionslösung Nukleinsäuren, Proteine, Naturstoffe, interkalierenden Farbstoffe, Enzyme, Zusatzstoffe, Viren, Prokaryoten, Eukaryoten, Fragmente aus synthetischen Molekülen und/oder Biomolekülen umfasst.

Die Reaktionslösung enthält gemeinsam mit den Reaktionskomponenten bevorzugt die Gesamtheit aller Moleküle, die es erlauben, von einem Zielmolekül, insbesondere einem DNA-Probenfragment, Amplifikate herzustellen. Die Zusammensetzung der Reaktionslösung und der Reaktionskomponenten legt in eindeutiger Weise fest, welche Zielmoleküle oder welche Abschnitte von Zielmolekülen in welcher Weise amplifiziert werden. Die Reaktionslösung umfasst bevorzugt Einzelbausteine für die Synthese der Amplifikate, ein Energiesystem und/oder ein Synthesesystem. Vorzugsweise ist eine DNA-Polymerase, insbesondere eine Pfu oder Taq Polymerase und dNTPs oder ein Mix aus dATPs, dCTPs, dGTPs, dTTPs und dUTPs und insbesondere ein UNG-Verdausystem enthalten. Des Weiteren können bevorzugt reaktionsverbessernde Komponenten in der Reaktionslösung vorliegen Dabei handelt es sich vorzugsweise um "bovine serum albumin" (BSA), Agarose, Tween, ultrageschallte Nukleinsäuren und/oder ein UNG-Verdausystem. Es kann auch bevorzugt sein, dass die Reaktionslösung eine RNA-Polymerase enthält. Es kann sich insbesondere auch um ein handelsüblicher PCR Amplifikationsmix handeln in welchen die Zielmoleküle gegeben werden.

Die Zielmoleküle können Poly-Nukleinsäuren, wie DNA-, cDNA oder RNA-Moleküle, aber auch Proteine, Antikörper, synthetische organische Moleküle oder Naturstoffe sein. Wenn es sich bei den Zielmolekülen nicht um Poly-Nukleinsäuren handelt, können diese direkt oder indirekt über ein eingebautes Label aus DNA oder RNA, amplifiziert und/oder derivatisiert werden. Im üblichsten Fall handelt es sich bei den Zielmolekülen um DNA. Die DNA-Zielmoleküle können unter anderem aus Viren, Pro- und/oder Eukaryoten gewonnen werden und/oder synthetischer Natur sein. Die Länge eines Zielmoleküls kann zwischen 1 und 10^10 Einzelmonomeren betragen. Solche Zielmoleküle können auch nach verschiedenen Verfahren in einzelne Fragmente unterteilt werden. Die Fragmente können außerdem einzel- oder doppelsträngig sein. Im Sinne der Erfindung können die Zielmoleküle auch als Proben oder Targets bezeichnet werden.

Wenn das Zielmoleküle keine DNA oder RNA ist, ist es bevorzugt, dass das Zielmolekül über ein eingebautes Label aus DNA oder RNA verfügt. Dieses Label kann dann über das beschriebenen Verfahren nachgewiesen werden.

Es ist bevorzugt, dass die Zielmoleküle an mindestens einem Ende Adaptoren mit einer einheitlichen Sequenz aufweisen. Die bevorzugte Länge der Adaptoren liegt zwischen 1 bis 1000 Einzelmolekülen. Die Adaptoren können unterschiedliche funktionelle Einheiten aufweisen. Adaptoren sind bevorzugt am Anfang und Ende eines Zielmoleküls in Form von einheitlichen und eindeutigen Sequenzen vorhanden. Sie definieren diejenigen Bereiche der Zielmoleküle, die amplifiziert werden. Die Adaptoren fungieren insbesondere für eine PCR als Primer. Mittels der Adaptoren können entweder alle oder nur bestimmten Untereinheiten der Gesamtheit aller Zielmoleküle amplifiziert werden. Weiterhin können die Adaptoren vorzugsweise mehrere unterschiedliche funktionelle Einheiten hintereinander besitzen, die insbesondere Bindestellen für RNA-Polymerasen oder zellfreie Expressionssysteme sein können. Die Adaptorsequenzen können beispielsweise aus gängigen Sequenziersystemen stammen (unter anderem Applied Biosystems/Life Technologies, Ion Torrent/Life Technology, Roche, Illumina, George Church/Dover Systems) und hierbei sequenzidentisch sein oder weitere Sequenzen oder Modifizierungen enthalten. Damit können die Adaptorsequenzen eine sequenzierfähige Oberfläche schaffen. Die Oberfläche ist sequenzierfähig, wenn an die Fängermoleküle amplifizierte Zielmoleküle gebunden haben. Die Amplifikate stehen dann für weitere Untersuchungen zur Verfügung.

Es ist bevorzugt, dass eine zu analysierende Probe insbesondere vor Einbringung in die Grundfläche verdünnt wird. Durch das Einbringen der Probenlösung in die Grundfläche wird die Probe auf viele Kavitäten verteilt, was dem Fachmann auch als "Aliqotieren" bekannt ist.

Produktmoleküle sind im Sinne der Erfindung bevorzugt solche Moleküle die durch die Amplifikation und/oder Derivatisierung der Zielmoleküle während des erfindungsgemäßen Verfahrens entstehen. Über den Nachweis der Produktmoleküle kann so auch das Vorhandensein der Zielmoleküle nachgewiesen werden.

Es ist bevorzugt, dass 1 bis 10^10 Produktmoleküle, bevorzugt Amplifikate generiert werden.

Die Reaktionslösung kann unter anderem über Zentrifugation, Vakuum, Zerstäuben, Eintauchen, Dipcoaten oder Bestreichen in die Kavitäten der Grundfläche eingebracht werden. Je nach Anzahl der in der Reaktionslösung eingebrachten Zielmoleküle werden in die Vorrichtung zwischen 1 und 10^10 Zielmoleküle eingebracht. Weiterhin ist bevorzugt, dass jede Kavität zwischen 1 und 10 Zielmoleküle enthält.

Es ist auch möglich, dass die Reaktionslösung die Reaktionskomponenten enthält. Alternativ können die Reaktionskomponenten in der Vorrichtung, der Abdeckvorrichtung und/oder der Grundfläche, vorgelagert werden. Diese Vorlagerung kann in vielfältiger Weise erfolgen.

Besonders bevorzugt ist dabei, dass die Reaktionskomponenten in einer Matrix bereitgestellt werden, bevorzugt einem Gel, besonders bevorzugt einem Agarosegel und wobei die Reaktionskomponenten bei Zugabe der Reaktionslösung und/oder Wärmezufuhr freigegeben werden.

Insbesondere eine Vorlagerung mittels eines Gels oder einer gelartigen Matrix, die sich erst unter Erhitzen auflöst ist sehr vorteilhaft. So kann zunächst die Reaktionslösung mit den Zielmolekülen eingefüllt werden, ohne dass Primer oder Detektionssonden verschleppt werden. Insbesondere die Verwendung von low melting Agarose ist hierzu bevorzugt. Da für die PCR ohnehin erhitzt werden muss, werden durch das Erhitzen zu Beginn des Amplifikationsschritts die Reaktionskomponenten freisetzen. Des Weiteren können in dieser Matrix auch die Polymerase, sowie alle Chemikalien für Detektion und/oder Amplifikation vorgelagert werden. In diesem Fall muss die Reaktionslösung außer den Zielmolekülen keine weiteren Reagenzien enthalten, da alle notwendigen Reagenzien werden als Reaktionskomponenten vorgelagert werden. Es ist aber auch möglich, dass nur ein Teil der Reagenzien, zum Beispiel die Primer oder Sonden, als Reaktionskomponenten vorgelagert werden und die anderen Reagenzien zusammen mit den Zielmolekülen in der Reaktionslösung enthalten sind und nachträglich auf die Vorrichtung gegeben werden.

Die Kavitäten der befüllten Grundfläche werden bevorzugt durch die Beaufschlagung mit einer Abdeckvorrichtung kompartimentiert beziehungsweise voneinander isoliert. Dadurch werden gewünschte Reaktion ermöglicht und ungewünschte Reaktionen oder Inhibitionen unterbunden.

Weiterhin können die Kavitäten aus einem Multilagenverbund oder track etched Membranen bestehen. Die Grundfläche kann ein Multifaserarray sein dessen Grundeinheit optische Fasern sind. Die Oberfläche kann eine 3-dimensionale Mikrostruktur sein, die einzelne Bereiche enthält, die durch geeignete Prozessführung voneinander zu Kompartimenten abtrennbar ist, z.B. kleine Säulen, an deren Spitze die Amplifikation abläuft. Das Material ist vorzugsweise kompatibel zu PCR Reaktionen und/oder zu anderen DNA-/RNA-Amplifikations-Methoden, sodass keine Inhibition oder Kontamination stattfindet. Es ist bevorzugt, dass das Material massenproduktionskompatibel hergestellt werden kann.

Es ist bevorzugt, dass die Grundfläche aus halbleitenden, amorphen, kristallinen, quasi-kristallinen und/oder faserartigen Material gefertigt ist. Diese Materialien haben sich als vorteilhaft erwiesen, da hierdurch stabile Grundflächen kostengünstig hergestellt werden können. Vorzugsweise können die Kavitäten einer Grundfläche unter anderem durch Ätzverfahren realisiert werden, wie zum Beispiel durch Trockenätzen/Reaktive Verfahren (unter anderem DRIE, Bosch-Prozess, ICP), nasschemische Verfahren (unter anderem Laugen, Säuren, HF) oder physikalischen Methoden (unter anderem Bohren, Sputtern, ionisches Ätzen oder Abformprozesse wie Spritzgießen und "Hot-Embossing", LIGA Verfahren, Mikropräzisionsfräsen). Die Grundfläche und/oder die Abdeckvorrichtung können bevorzugt beliebige Beschichtungen bevorzugt mit Biomolekülen (DNA, RNA, Proteine), Metallen, Metalloxiden oder Kunststoffen enthalten.

Die Abdeckvorrichtung kann entweder ein DNA Mikroarray oder ein einfaches Substrat sein. Es ist außerdem bevorzugt, dass die Abdeckvorrichtung aus halbleitenden amorphen, kristallinen und/oder faserartigen Materialien, besonders bevorzugt Glas, Polymer, PDMS, PP, COC oder COP besteht. Weiterhin kann die Abdeckvorrichtung ein Verbund aus zwei Materialien sein. Das Substrat kann auch eine Klebefolie sein, bevorzugt eine PCR taugliche Klebefolie für Mikrotiterplatten. Weiterhin kann ein Substrat mit einem weiteren Material beschichtet sein (z.B. mit PDMS). Dabei ist darauf zu achten, dass das Material kompatibel zu den PCR Reaktionen ist und keine Inhibition oder Kontamination verursacht.

Dem Fachmann ist bekannt, dass ein DNA Mikroarray Reagenzien, bevorzugt DNA Sequenzen umfasst, die vorzugsweise in Form von Spots vorliegen. Es kann bevorzugt sein, dass auf der Abdeckvorrichtung oder in den Kavitäten ein DNA Mikroarray vorliegt, wobei es auch bevorzugt sein kann, dass auch weitere Komponenten, die für eine PCR benötigt werden, in Form von Spots oder Flächen auf die Abdeckvorrichtung oder in die Kavitäten gebracht werden.

DNA-Mikroarrays dienen insbesondere dazu, die mRNA-Menge bestimmter Gene oder rRNA bestimmter Organismen nachzuweisen. Es existieren im Wesentlichen zwei verschiedene Arten von DNA-Mikroarrays, einerseits solche, bei denen cDNA, Oligonukleotide oder Fragmente von PCR-Produkten die der mRNA entsprechen auf das Trägermaterial gedruckt werden ("Spotted Mikroarrays") und solche, die auf synthetisch hergestellten Oligonukleotiden beruhen ("Oligonukleotide Mikroarrays"). Diese dienen als Sonden, die an definierte Positionen eines Rasters z. B. auf Glasträger aufgebracht werden.

Die Reaktionskomponenten können wahlweise auf der Abdeckvorrichtung aufgebracht sein, oder bereits direkt auf die Grundfläche, bevorzugt in den Kavitäten, gespottet sein. Durch diese Vorlagerung bestimmter Reaktionskomponenten wird somit ein DNA-Array entweder durch die Abdeckvorrichtung oder durch die Grundfläche selbst realisiert.

Es ist bevorzugt, dass Sonden, besonders bevorzugt unterschiedliche Sonden, als Reaktionskomponenten in den Kavitäten der Grundfläche vorgelagert werden. Gleichzeitig wird eine Art von Primern in der Abdeckvorrichtung vorgelagert. Das Multiplexen wird in diesem Fall durch die unterschiedlichen definierten Bereiche der Grundfläche ermöglicht.

Die Zielmoleküle können an eine Oberfläche der Grundfläche und/oder der Abdeckvorrichtung gebunden werden. Es ist auch möglich, dass die Zielmoleküle lose in Form eines Arrays lokalisiert bereit gestellt werden.

Die Grundfläche selbst kann DNA Mikroarray ähnliche Eigenschaften aufweisen, das heißt die oben aufgeführten Eigenschaften besitzen. Für ein bevorzugtes erfindungsgemäßes Verfahren wird die Grundfläche mit einer Abdeckvorrichtung, entweder mit einem Mikroarray oder mit einem Substrat, das keine aktiven Komponenten für die Reaktion enthält (z.B. einfacher Polymerchip in Format eines Mikroarrays) verschlossen.

Wenn ein Mikroarray als Abdeckvorrichtung verwendet wird, kann dieses mit allen gängigen Verfahren hergestellt sein. Dies sind bevorzugt Verfahren der Firmen Affymetrix, Agilent, LC Sciences, Combimatrix, Nimblgen, Febit, Biofluidix. Es ist weiterhin bevorzugt, dass die Primer synthetisiert werden (z.B. von Biomers, IBA, IDT) und dann mit unterschiedlichen Spotting Verfahren (Nadeldrucker, Pinprinter, Topspot® System) auf das Substrat des Mikroarrays aufgebracht werden.

Bevorzugt ist weiterhin, dass die Grundfläche zusätzlich einen Bereich zur Bereitstellung der Reaktionslösung aufweist. Es ist dabei bevorzugt, dass die Reaktionslösung auf den Bereich zur Bereitstellung der Reaktionslösung gegeben wird und durch das Auflegen der Abdeckvorrichtung die Reaktionslösung auf die Kavitäten verteilt wird. Es war völlig überraschend, dass durch die erfindungsgemäße Konstruktion der Grundfläche eine Befüllung der Kavitäten erreicht wird, ohne dass hierfür Energie oder weitere Arbeitsschritte aufgewendet werden müssten. Bevorzugt wird eine Reaktionslösung enthaltend die Zielmoleküle in oder auf einen bestimmten Bereich der Grundfläche gebracht, der für die Aufnahme einer Reaktionslösung vorgesehen ist. Anschließend kann die Abdeckvorrichtung mit einem Ende auf diesen Bereich aufgesetzt und umgeklappt oder aufgeschoben und auf die Grundfläche gebracht werden, so dass die Abdeckvorrichtung innerhalb der Flüssigkeits-Barriere angeordnet wird. Weiterhin kann die Abdeckvorrichtung senkrecht aufgedrückt werden, oder zunächst schräg angedrückt werden und dann von einer Kante zur gegenüberliegenden Kante wie ein Deckel zugeklappt werden oder waagerecht über die Grundfläche gezogen werden (slippen) beziehungsweise geschoben werden. Im Falle des Klappens wird die Reaktionslösung über den hip hinweg verdrängt und kann so alle Kavitäten befüllen. Im Falle des Schiebens wird die Reaktionslösung an der Vorderkante vorangeschoben und befüllt so die Grundfläche bis sie komplett aufgebraucht ist.

Die Grundfläche ist so gestaltet, dass eine Reaktionslösung ohne oder mit nur sehr geringem Totvolumen eingefüllt werden kann. Dies wird unter anderem durch die Barrieren am Rand der Grundfläche realisiert sowie durch eine Geometrie der Kavitäten die eine Selbstbefüllung ermöglicht. Die Concus-Finn Bedingung sind bevorzugter Weise erfüllt.

Es ist außerdem bevorzugt, dass die Grundfläche zusätzlich ein Reservoir zur Aufnahme von überschüssiger Reaktionslösung aufweist. Durch diese Ausführungsform wird ein sauberes Arbeiten ermöglicht und die allgemeine Gefahr von Kontaminationen im Arbeitsbereich verringert.

Es ist bevorzugt, dass die Abdeckvorrichtung nach dem zueinander Anordnen von Grundfläche und Abdeckvorrichtung die Kavitäten bedeckt, wobei es auch vorteilhaft sein kann, dass die Abdeckvorrichtung nicht alle Kavitäten, sondern nur eine Auswahl dieser bedeckt. Die Reaktionslösung, bei der es sich vorzugsweise um eine hydrophile Flüssigkeit handelt, die eine zu analysierenden Zielmoleküle umfasst, wird bevorzugt selbstständig in die Kavitäten befördert. Es ist nicht nötig, die Reaktionslösung mit einer Befüllvorrichtung (z. B. einer Pipette) in die Kavitäten einzubringen. Dies stellt einen erheblichen Vorteil zum Stand der Technik dar. Das heißt, es wird durch die Erfindung möglich, Reaktionskomponenten in den Kavitäten und/oder auf der Abdeckvorrichtung vorzulagern, so dass lediglich die Zielmoleküle und eventuell weitere Reagenzien für die Amplifikationsreaktion oder die Derivatisierungsreaktion eingebracht werden müssen. Die Reaktionskomponenten können hierbei unterschiedliche Bestandteile und Funktionen besitzen, wodurch in einer Grundfläche z. B. unterschiedliche Bereiche einer Zielsequenz mittels einer digitalen PCR analysiert werden können. Auf einer Grundfläche können demnach unterschiedliche Reaktionen durchgeführt werden, wodurch das "Multiplexen" möglich wird und was zusätzlich den Arbeitsaufwand und die Kosten erheblich reduziert.

Weiterhin ist bevorzugt, dass die Zielmoleküle und/oder die Produktmoleküle anschließend analysiert, sequenziert und/oder derivatisiert werden. Bevorzugt wird dabei die Vorrichtung wieder in Abdeckvorrichtung und Grundfläche getrennte. Der Teil der Vorrichtung auf dem Reaktionskomponenten vorgelagert waren, bevorzugt Sonden, steht dann für weitere Analyseverfahren zur Verfügung.
In einer weiteren bevorzugten Ausführungsform betrifft die Erfindung eine Vorrichtung zur Durchführung eines der genannten Verfahren,
wobei
die Vorrichtung eine Grundfläche mit Kavitäten und
eine Abdeckvorrichtung umfassend Spots umfasst, und wobei
die Abdeckvorrichtung bevorzugt ein Mikroarray ist, und wobei
die Abdeckvorrichtung Fängermoleküle umfasst, welche an der Abdeckvorrichtung immobilisiert sind, wobei
die Grundfläche und die Abdeckvorrichtung so zueinander angeordnet sind, dass Reaktionsräume entstehen
dadurch gekennzeichnet, dass
die Grundfläche eine Flüssigkeits-Barriere aufweist, welche die Kavitäten umrandet, wobei die Flüssigkeits-Barriere bevorzugt eine chemische und/oder physikalische Barriere ist und/oder
die Grundfläche zusätzlich einen Bereich zur Bereitstellung der Reaktionslösung aufweist und/oder die Grundfläche zusätzlich ein Reservoir zur Aufnahme von überschüssiger Reaktionslösung aufweist
und wobei ein Spot der Abdeckvorrichtung mehrere Kavitäten der Grundfläche abdeckt.

Es ist dabei besonders bevorzugt, dass die Vorrichtung zusätzlich eine Kassette umfasst, welche die Grundfläche und die Abdeckvorrichtung aufnehmen kann und in einen die Reaktion begünstigenden Zustand bringt. Weiterhin kann diese Vorrichtung eine Befüllvorrichtung zur Verfügung stellen. Diese gesamte Vorrichtung verhindert weiterhin eine Kontamination mit Reaktionslösung und Reaktionskomponenten. Im Fall des Amplifikationssystems PCR gewährleistet die Vorrichtung einen guten Wärmeeintrag. Im Fall der mechanischen Verdeckelung hält diese einen engen mechanischen Kontakt zwischen der Grundfläche und der Abdeckvorrichtung während der Reaktion.

Es ist besonders bevorzugt, dass die beschriebenen besonderen Ausführungsformen des Verfahrens in der Vorrichtung umgesetzt wurden. Besonders bevorzugt ist, dass die Grundfläche der Vorrichtung eine Flüssigkeits-Barriere aufweist, welche die Kavitäten umrandet, wobei die Flüssigkeits-Barriere bevorzugt eine chemische und/oder physikalische Barriere ist.

Ein immenser Vorteil der Erfindung liegt in der Reduktion des Reaktionsvolumens, wodurch die generellen Kosten gesenkt werden und die Kontaminationsgefahr minimiert sowie der Durchfluss erhöht werden kann. Die Erfindung kann daher beispielsweise für die Feststellung von Mutationen in der nicht-invasiven Diagnose eingesetzt werden, da hier eine hochauflösende Analyse einzelner Moleküle und Amplifikationen dieser einzelnen Moleküle erforderlich ist. Es ist bevorzugt, dass jeweils in einem Reaktionsvolumen beziehungsweise in einer Kavität ein Zielmolekül bevorzugt eine DNA-Sequenz vorliegt. So wird verhindert, dass spezielle Sequenzen bei der PCR bevorzugt werden und die Bildung von unerwünschten Primer-Dimeren wird reduziert. Die Erfindung kann besonders bevorzugt zur Detektion von Punktmutation-bezogenen Krankheiten eingesetzt werden. So erlaubt zum Bespiel die Kombination aus digitaler PCR und Anreicherung von fetaler DNA unter parentaler DNA eine einfache Detektion von Erkrankungen die durch Punktmutationen hervorgerufen werden. Im Stand der Technik ist bisher beispielsweise nicht beschrieben, dass die Detektion von Aneuploidie mit Systemen zur Anreicherung fetaler DNA unter parentaler DNA möglich ist. Die Erfindung liefert somit durch die Kombination einer Technik zur Voranreicherung fetaler DNA und die nachgeschaltete Analyse dieser in einem multiplexen erfindungsgemäßen PCR-System erstmalig ein erfolgreiches System, welches zur nicht-invasiven Detektion von Kinderkrankheiten verwendet werden kann.

Ein weiter bevorzugter Anwendungsbereich des erfindungsgemäßen Verfahrens ist der Nachweis von Single Nukleotide Polymorphism (SNP) und Genaberrationen. Hierzu werden Primer und Sonden so generiert, dass spezifisch nur eine Sequenz mit dem jeweiligen SNP erweitert wird. Die Detektion kann mittels eines Farbcodes (also z.B. A = grün, C = rot, G = blau, T = tiefrot) oder durch räumliche Trennung von vier einzelnen Spots erfolgen. Dabei erlauben positive Spots eine Quantifizierung. Vorzugsweise spricht nur ein Spot an (homozygote Vererbung des SNPs, Mutter und Vater identisch) oder genau zwei, die vorzugsweise ungefähr gleich viele positive Signale liefern (heterozygote Vererbung 50% Vater 50% Mutter). Mittels dieses Verfahrens lassen sich auch Genaberrationen wie Trisomien (2/3 Spot 1 und 1/3 Spot 2 oder je 3 Spots mit je 1/3 Signal), Duplikationen oder Deletionen nachweisen.

Außerdem ist bevorzugt, dass das erfindungsgemäße Verfahren zum Nachweis von bestimmten Nukleinsäuresequenzen (Genotyping) eingesetzt wird. Hier werden auf dem jeweiligen Spot die passenden Primer und eine Sonde vorgelagert wie z.B. Resistenzgene und interne "house-keeping Gene". Positive Signale erlauben eine Quantifizierung. Der relative Vergleich zwischen House-keeping Genen und Resistenzen erlaubt eine Aussage, wie viele und welche der getesteten Genome eine Resistenz aufweisen.

Es ist außerdem bevorzugt, dass das Verfahren für die Quantifizierung einer oder mehrerer DNA Konzentration(en) verwendet wird. Bei dieser Ausführungsform wird eine Volumenserie in Grundfläche eingebracht, sodass die Kavitäten mit unterschiedlichen Volumen befüllt sind. Außerdem wird ein allgemeines Primer und Sondensystem in die Grundfläche eingebracht. Es wird nur die Probe hinzugegeben. Die Oberfläche kann homogen mit dem Primersystem beschichtet sein, oder einige Streifen/Spots an Referenzgenen enthalten. Aufgrund der geringer werdenden Volumina wird irgendwann ab einem bestimmten Volumen im Mittel genau ein oder weniger DNA-Stränge pro Kavität vorhanden sein. Hier findet nun eine Digitale PCR statt. Anhand der nicht mehr nur positiven Kavitäten lässt sich dann eine Bestimmung der DNA-Konzentration ermitteln.

Es ist auch bevorzugt, dass mehrere unterschiedliche Spezies/Subtypen "digitalisiert" werden. Dies erlaubt z.B. aus einer E.coli-Population die Gesamtzahl der Bakterien und die Anzahl der schädlichen Subtypen z.B. *EHEC* etc. in einem einzigen Ansatz zu bestimmen. Die Digitalität wird hier nicht dadurch erreicht, dass weniger Zielmoleküle als Kavitäten vorhanden sind, sondern bevorzugt dadurch, dass in den Kavitäten Zielmoleküle unterschiedlicher Subtypen amplifiziert und detektiert werden.

Es ist außerdem ein Vorteil der Erfindung, dass mit dem Verfahren alle bekannten Anwendungen der digitale PCR abgedeckt werden können.

In einer weiteren bevorzugten Ausführungsform betrifft die Erfindung einen Kit, umfassend eine Grundfläche und eine Abdeckvorrichtung, sowie die Reaktionslösung (ohne Probe). Es ist dann nur noch notwendig die Zielmoleküle in die Reaktionslösung ohne Probe hinzuzugeben und die fertige Reaktionslösung auf die Grundfläche aufzutragen. Die Erfindung betrifft demgemäß auch ein Kit zur Durchführung einer dPCR. Es war völlig überraschend, dass ein Kit hergestellt werden konnte, mit dem sich das erfindungsgemäße Verfahren durchführen lässt. Es war dabei nicht zu erwarten, dass die Vorrichtung mit den vorgelagerten Reaktionskomponenten dauerhaft gelagert werden kann, ohne dass die Reaktionskomponenten ihre Funktion verlieren. Das erfindungsgemäße Kit kann somit über einen langen Zeitraum gelagert werden und erleichtern daher die Durchführung des erfindungsgemäßen Verfahrens um ein Vielfaches.

Mit dem erfindungsgemäßen Verfahren können bis zu 10⁶ Targets detektiert werden. Die Anzahl hängt dabei von der Größe der einzelnen Kavitäten und der Größe der Spots auf dem Array ab.

Es ist ein Vorteil der Erfindung, dass nur Standard-Geräte benötigt werden. Zum Beispiel können für die PCR bereits kommerziell erhältliche Geräte wie z.B. für Objektträger (Slide-Cycler) oder als Einsatz für ein Mikrotiterplatten PCR Cycler verwendet werden. Für die Detektion kann z. B. ein Standard-Gerät für das Auslesen von DNA Mikroarrays verwendet werden (Array-Scanner).

Durch ein einfaches Neudesign der DNA-Sonden (Abänderung der DNA Sequenzen) auf dem Array kann das Detektionssystem auf andere Zielmoleküle zugeschnitten werden, so dass das Verfahren universell einsetzbar ist. Zudem ist die Sensitivität über die Festlegung der Anzahl an Kavitäten pro Spots definierbar.

Die Kavitäten der Grundfläche können innerhalb einer Grundfläche unterschiedliche Größen aufweisen, weshalb eine Reaktionslösung ohne vorherige Quantifizierung untersucht werden kann. Durch geschickte Wahl der Reaktorvolumina, wird sich immer ein Bereich finden, der "digital" ist. Und die anderen Bereiche können mittels der MPN-Methode Zusatzinformationen liefern. Durch eine Vervielfachung der Kammergrößen kann ein großer Konzentrationsbereich einer Probenlösung abgedeckt werden. Dadurch können aussagekräftige Ergebnisse erzielt werden, ohne vorherige Quantifizierung der DNA in der Probe. Dadurch werden Zeit und Handhabungsschritte eingespart.

Die Signale werden bevorzugt digital ausgelesen, so dass lediglich das Endergebnis der Analyse ausgewertet werden muss und hierfür Reaktions- und Detektionsvorrichtung gekoppelt werden können. Hierdurch können der aparative Aufwand minimiert werden.

Die Grundfläche kann z. B. durch Spritzgießen oder Heißprägen hergestellt werden, wohingegen für die Abdeckvorrichtung existierende, über Jahrzehnte hinweg etablierte Produktionsanlagen zur Aufbringung der Reaktionskomponenten nutzbar sind. Somit ist die Grundfläche mittels günstiger Herstellungsverfahren bereitstellbar.

Im Gegensatz zu einigen Verfahren des Standes der Technik, die Öl für die Erzeugung von Reaktionskompartimenten verwenden, findet bei dem Verfahren die Aufteilung einer Probe in viele Kavitäten mechanisch (ohne Öl), bevorzugt mithilfe der Abdeckvorrichtung statt. Dadurch werden alle Nachteile umgangen, die im Stand der Technik beschrieben sind.

Vorteilhafterweise kann das Produktmolekül, bevorzugt das Amplifikat, in der Grundfläche und/oder auf der Abdeckvorrichtungs-Oberfläche gebunden werden und erlaubt so, falls benötigt oder gewünscht, auch eine spätere detaillierte Analyse wie z.B. Sequenzierungen oder die Rückgewinnung des entsprechenden Genmaterials (z. B. Rückgewinnung des generierten PCR Produktes, Möglichkeit der Sequenzierung eines DNA Mikroarrays nach der Reaktion).

Die anmeldungsgemäße Lehre zeichnet sich außerdem insbesondere durch folgende Merkmale aus:
- Vorliegen eines seit langem ungelösten dringenden Bedürfnisses für die Lösung des mit der Erfindung gelösten Problems
- die Einfachheit der Lösung spricht für erfinderische Tätigkeit, insbesondere da sie kompliziertere Lehren ersetzt
- Verbesserung, Leistungssteigerung, Verbilligung, Ersparnis an Zeit, Material, Arbeitsstufen und Kosten,
- erhöhte Zuverlässigkeit, Beseitigung von Fehlern, Qualitätshebung, Wartungsfreiheit, größere Effektivität, höhere Ausbeute,

Insbesondere die vorteilhaften Ausführungsformen der Erfindung weisen mindestens einen oder mehrere der genannten Vorteile auf.

### BEISPIELE

Im Folgenden soll die Erfindung anhand von Figuren und einem Beispiel erläutert werden, ohne jedoch auf diese beschränkt zu sein. Es zeigen:
- Fig. 1.1 und 1.2: Ausführungsform eines bevorzugten Verfahrens und der entsprechenden Vorrichtung
- Fig. 2A, 2B: Bevorzugtes Design einer Vorrichtung
- Fig. 3: Bevorzugtes Verfahren unter Verwendung einer dPCR
- Fig. 4: Immobilisierung von PCR Primern und anschließende Festphasen PCR an einem Modellsystem

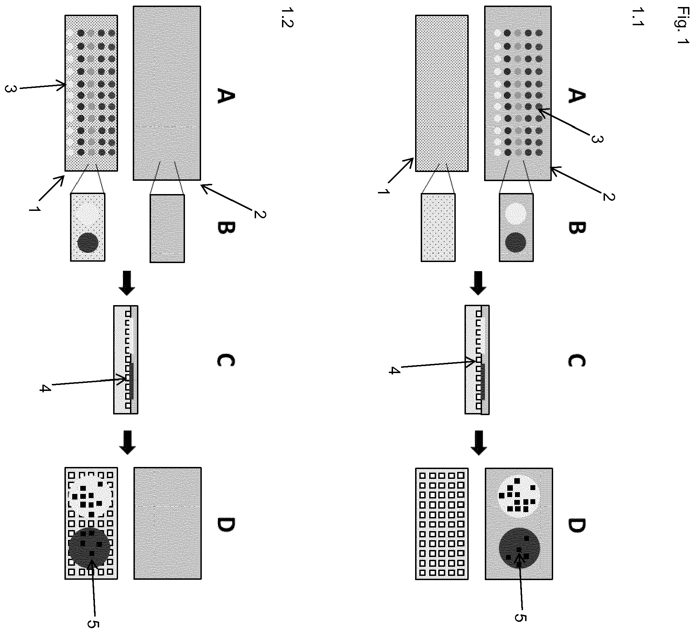

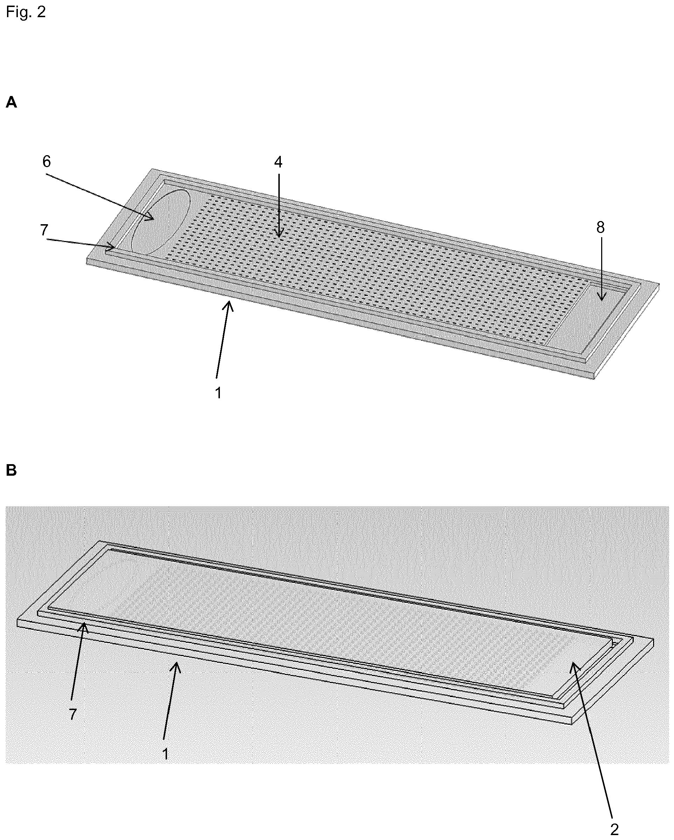

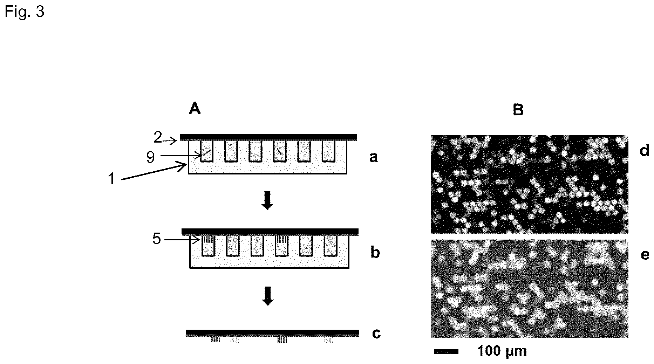

Für die Festphasen PCR ist es eine Voraussetzung, entsprechende Oberflächen mit PCR-Primern zu beschichten, so dass der PCR-Primer für eine Verlängerung durch eine DNA-Polymerase zugänglich/verfügbar ist. Es wurden sowohl die Oberfläche der Grundfläche als auch die Oberfläche der Abdeckvorrichtung mit PCR-Primern beschichtet (Fig. 3a). Bei der Abdeckvorrichtung handelt es sich vorzugsweise PDMS Träger. Eine DNA Zielsequenz wurde mit einem PCR-Reaktionsmix vermischt und in die Grundfläche eingebracht. Die Grundfläche wurde mit der Abdeckvorrichtung, in diesem Fall einem DNA-Mikroarray bedeckt (Fig. 2). Um eine digitale PCR durchzuführen, wird die DNA-Zielsequenz verdünnt, so dass jede einzelne Kavität entweder ein oder kein DNA-Molekül umfasst. Durch die anschließende PCR-Reaktion wird in jeder Kavität, welche ursprünglich ein DNA-Molekül enthielt, ein PCR Amplifikationsprodukt generiert und insbesondere an die Oberfläche der Grundfläche gebunden (Fig. 1). Da in dem vorliegenden Beispiel aber auch die Abdeckvorrichtung mit Festphasen-Primern versehen wurde, wurden die DNA-Amplifikate ebenfalls an der Abdeckvorrichtung immobilisiert. Hierdurch wird eine exakte Kopie der Verteilung der Produktmoleküle der Grundfläche am Mikroarray (Abdeckvorrichtung) bereitgestellt (Fig. 1D). Um die Amplifikate zu visualisieren, werden auf PTP (Pikotiterplatten) und der Abdeckvorrichtung eine Hybridisierung durchgeführt.

Eine für die für Festphasen PCR geeignete Immobilisierung von PCR Primern konnte in voller Funktionalität auf Grundflächen aus GS FLX Titaniumchips, GS FLX-Chips und außerdem auf typischen Materialien die in der Mikrofluidik verwendet werden. Hierzu zählen Glas, Polydimethylsiloxan (PDMS), Cyclo-Olefin-Polymer (COP), Cyclo-Olefin-Copolymere (COC), und Polypropylen (PP) nachgewiesen werden.

Für die Durchführung des Verfahrens bei Digitalität wurden << 1 × 10^5 Zielmoleküle in 1 × 10^5 Kavitäten auf eine 13 × 13 mm² großen Grundfläche gegeben. Als Grundfläche wurde ein GS FLX Titanium-Chip verwendet. Nach 50 Thermozyklen wurden die Signale auf der Grundfläche und der Abdeckvorrichtung automatisch gezählt. Als Abdeckvorrichtung wurde ein PDMS-Träger verwendet. Wenn eine digitale Festphasen-PCR mit zwei unterschiedlichen DNA-Sequenzen durchgeführt wurde, wurde ein zweifarbiges Bild nach der Hybridisierung (Figur 3, b) generiert. Hierbei wurde stets eine gute Vergleichbarkeit von Grundfläche und Abdeckvorrichtung erzielt.

Dieser Versuch ist ein Beispiel für die parallele Analyse von Konzentrationen zweier unterschiedlicher DNA-Zielmoleküle. Für die Farben rot und grün kann die jeweilige Konzentration errechnet werden. Gelb zählt als rot und grün gleichzeitig. Die Gesamt-DNA-Konzentration für jedes Zielmolekül ist die Zahl der positiven Kavitäten geteilt durch die Zahl aller Kavitäten geteilt durch das Volumen einer Kavität.

**Figur 1.1** zeigt eine bevorzugte Ausführungsform der Erfindung. In (A) wird eine Abdeckvorrichtung **2** gezeigt, welche ein DNA Mikroarray ist. Darunter ist eine Grundfläche **1** mit Kavitäten **4** gezeigt, wobei die Kavitäten **4** mit einem PCR-Reaktionsmix enthaltend verdünnte DNA-Zielmoleküle gefüllt sind. (B) zeigt eine Vergrößerung der Spots **3** des Mikroarrays **2** beziehungsweise der Kavitäten **4**. In (C) wird gezeigt, wie die zusammengebaute Vorrichtung gezeigt. Abdeckvorrichtung **2** und Grundfläche **1** sind dabei so zueinander angeordnet, dass die Kavitäten **4** und die Spots **3** des Mikroarrays **2** mit einander in Kontakt kommen. In dem gezeigten Fall decken die Spots **3** des Mikroarrays **2** jeweils mehrere Kavitäten **4** der Grundfläche **1** ab. In diesem Schritt läuft auch die Amplifikationsreaktion ab. Ein Temperaturzyklenprotokoll wird verwendet und DNA-Amplifikate **5** werden hergestellt. Dabei entstehen Amplifikate **5** nur in den Kavitäten **4** in denen ein Zielmolekül eingebracht wurde. In dem gezeigten Ausführungsbeispiel werden die PCR-Produkte **5** (= Produktmoleküle im Sinne der Erfindung) an Fängermolekülen des Mikroarrays **2** kovalent immobilisiert. Der bevorzugte Mechanismus ist hier die Festphasen-PCR. Anschließend werden die Abdeckvorrichtung **2** und die Grundfläche **1** wieder voneinander getrennt. Die Abdeckvorrichtung **2** wird gewaschen und bevorzugt mit einer Zwei-Farben-Hybridisierung gefärbt und gescannt. In (D) wird das Ergebnis des Verfahrens gezeigt. Ein PCR-Produkt **5** von zwei unterschiedlichen DNA-Sequenzen (1500 und 350 Basenpaare lang) wird auf dem Mikroarray **2** detektiert.

**Figur 1****.****2** zeigt eine weitere bevorzugte Ausführungsform der Erfindung. In (A) wird eine Abdeckvorrichtung **2** gezeigt, welche ein einfaches Substrat ist. Darunter ist eine Grundfläche **1** mit Kavitäten **4** gezeigt, wobei die Kavitäten **4** mit einem PCR-Reaktionsmix enthaltend verdünnte DNA-Zielmoleküle gefüllt sind. Außerdem wurde ein Mikroarray auf die Grundfläche **1** gespottet, sodass die Reaktionskomponenten in der Grundfläche **1** vorgelagert wurden. Die Spots **3** sind dabei größer als die Kavitäten **4**, sodass immer mehrere Kavitäten **4** in einem Spot **3** liegen. (B) zeigt eine Vergrößerung der Spots **3** des Grundfläche **1** und der Kavitäten **4**. In (C) wird gezeigt, wie die zusammengebaute Vorrichtung gezeigt. Abdeckvorrichtung **2** und Grundfläche **1** sind dabei so zueinander angeordnet, dass die Kavitäten **4** durch die Abdeckvorrichtung **2** verschlossen wurden. In diesem Schritt läuft auch die Amplifikationsreaktion ab. Ein Temperaturzyklenprotokoll wird verwendet und DNA-Amplifikate **5** werden hergestellt. Dabei entstehen Amplifikate **5** nur in den Kavitäten **4** in denen ein Zielmolekül eingebracht wurde. In dem gezeigten Ausführungsbeispiel werden die PCR-Produkte **5** (= Produktmoleküle im Sinne der Erfindung) an Fängermolekülen des Grundfläche **1** kovalent immobilisiert. Der bevorzugte Mechanismus ist hier die Festphasen-PCR. Anschließend werden die Abdeckvorrichtung **2** und die Grundfläche **1** wieder voneinander getrennt. Die Grundfläche **1** wird gewaschen und bevorzugt mit einer Zwei-Farben-Hybridisierung gefärbt und gescannt. In (D) wird das Ergebnis des Verfahrens gezeigt. Ein PCR-Produkt **5** von zwei unterschiedlichen DNA-Sequenzen (1500 und 350 Basenpaare lang) wird auf der Grundfläche **1** detektiert.

**Figur 2** zeigt das mögliche Design einer Grundfläche **1** im Sinne der Erfindung. Die gezeigte Grundfläche **1** zeigt einen Bereich **6** in den die zu untersuchende Reaktionslösung eingegeben wird. Nach Befüllung der Kavitäten **4** und Verschluss mit einer Abdeckvorrichtung **2** ist die Abdeckvorrichtung **2** umfasst von einer erhabenen Barriere **7**. Die Vorrichtung verfügt außerdem über ein Reservoir **8** das überschüssiges Probenvolumen fasst.

**Figur 3****:** In (a) wird eine Grundfläche **1** gezeigt, die mit einem PCR-Reaktionsmix enthaltend verdünnte DNA-Zielmoleküle **9**, gefüllt ist und mit einer Abdeckvorrichtung **2** verschlossen wurde. Während der Durchführung einer Amplifikationsreaktion wird ein PCR-Produkt **5** in den Kavitäten **4** mit Zielmolekülen **9** hergestellt. Das PCR-Produkt **5** bindet kovalent an immobilisierte DNA-Fängermoleküle, in diesem Fall Festphasenprimer, sowohl an der Grundfläche **1** als auch an der Abdeckvorrichtung **2**. Die Abdeckvorrichtung **2** und die Grundfläche **1** werden wieder voneinander getrennt und beide Oberflächen werden gewaschen und bevorzugt mit einer Zwei-Farben-Hybridisierung gefärbt und gescannt.

**Figur 3** (B) zeigt die Ergebnisse der digitalen PCR. Ein PCR-Produkt **5** von zwei unterschiedlichen DNA-Sequenzen (1500 und 350 Basenpaare lang) wird auf der Abdeckvorrichtung **2** (d) und auf der Grundfläche **1** (e) detektiert. Die drei Farben rot (dunkel), grün (mittel) und gelb (hell) zeigen die positiven Kavitäten **4** beziehungsweise das "Spiegelbild" der positiven Kavitäten **4** auf der Abdeckvorrichtung **2**.

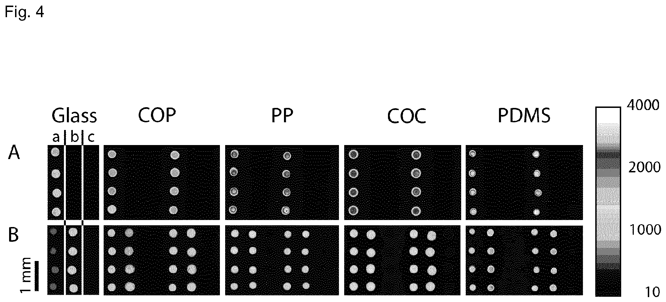

**Figur 4** zeigt Cy5 Scans von DNA Mikroarrays enthaltend einen Cy5-gelabelten Primer als nicht verlängerbare Spotting-Kontrolle (Spalte a), einen verlängerbaren Primer als Verlängerungskontrolle (Spalte b), und einen ungelabelten und nicht verlängerbaren Primer als Negativkontrolle (Spalte c). Pro Reaktionslösung wurden vier Reihen von Spots verwendet. Die Arrays wurden vor (Reihe A) und nach (Reihe B) der Festphasen-PCR und Färbung gescannt. Hochspezifische Verlängerungen der verlängerbaren Primer in konnte bei allen Polymeren und auch auf Glas beobachtet werden.

Die beiden Ergebnisse zusammen (Figur 3 und 4) zeigen, dass es möglich ist zum einen eine digitale PCR durchzuführen, bei der das PCR Produkt an eine Festphase immobilisiert wird (Figur 3, B, d und e). Weiterhin wurde gezeigt, dass man mit Primern, immobilisiert im Format eines klassischen DNA Mikroarrays, eine Festphasen PCR durchführen kann (Figur 4). Die Kombination aus beiden Ergebnissen schafft eine bevorzugte Ausführungsform des neuen Verfahrens für die gemultiplexte digitale PCR. Diese ist im Stand der Technik werde bekannt noch nahegelegt.

### Bezugszeichenliste

- 1: Grundfläche
- 2: Abdeckvorrichtung
- 3: definierte Bereiche auf der Abdeckvorrichtung (Spots)
- 4: Kavitäten
- 5: Produktmoleküle
- 6: Bereich zur Aufbringung einer Probe
- 7: Barriere
- 8: Reservoir das eventuell überschüssiges Probenvolumen fasst
- 9: DNA Zielmolekül

## Patentansprüche

1. Verfahren zum Nachweis von mindestens einem Zielmolekül und/oder Produktmolekül,
umfassend die folgenden Schritte
a) Bereitstellen einer Grundfläche mit offenen Kavitäten und einer Abdeckvorrichtung, wobei
(i) auf der Grundfläche und/oder der Abdeckvorrichtung Reaktionskomponenten in definierten Bereichen vorhanden sind, wobei die definierten Bereiche auf der Grundfläche im Bereich der Kavitäten liegen und es mindestens zwei definierte Bereiche mit unterschiedlichen Reaktionskomponenten gibt, und
(ii) die Grundfläche eine Flüssigkeits-Barriere aufweist, welche die Kavitäten der Grundfläche umrandet, und einen Bereich zur Bereitstellung einer Reaktionslösung aufweist, und
(iii) die Abdeckvorrichtung Spots umfasst und ein Spot der Abdeckvorrichtung mehrere Kavitäten der Grundfläche abdeckt,
b) Zugabe der Reaktionslösung auf den Bereich zur Bereitstellung der Reaktionslösung, wobei die Reaktionslösung die Zielmoleküle enthält,
c) Auflegen der Abdeckvorrichtung auf die Grundfläche, so dass die Abdeckvorrichtung innerhalb der Flüssigkeits-Barriere angeordnet wird, wobei durch das Auflegen der Abdeckvorrichtung die Reaktionslösung auf die Kavitäten verteilt wird, die Reaktionslösung in die Kavitäten eindringt und nach dem Auflegen der Abdeckvorrichtung jede Kavität mit der Abdeckvorrichtung einen abgeschlossenen Reaktionsraum bildet,
d) Durchführen einer Amplifikationsreaktion, einer Nachweisreaktion und/oder einer Derivatisierungsreaktion, wobei die Reaktionslösung mit den Reaktionskomponenten in Kontakt kommt und Produktmoleküle entstehen, und
e) Nachweis von mindestens einem Zielmolekül und/oder Produktmolekül, wobei mindestens zwei unterschiedliche Abschnitte des Zielmoleküls und/oder Produktmoleküls in separaten abgeschlossenen Reaktionsräumen nachgewiesen werden und/oder mindestens zwei unterschiedliche Zielmoleküle und/oder Produktmoleküle in separaten abgeschlossenen Reaktionsräumen nachgewiesen werden.

2. Verfahren nach Anspruch 1,
**dadurch gekennzeichnet, dass**
die Abdeckvorrichtung ein Mikroarray ist und die definierten Bereiche Spots des Mikroarrays sind und/oder die definierten Bereiche in den Kavitäten der Grundfläche liegen.

3. Verfahren nach Anspruch 1 oder 2,
**dadurch gekennzeichnet, dass**
die Reaktionskomponenten Primer und/oder Sonden enthalten, und/oder die Reaktionskomponenten oder Teile davon an der Grundfläche und/oder der Abdeckfläche immobilisiert sind.

4. Verfahren nach mindestens einem der vorher genannten Ansprüche,
**dadurch gekennzeichnet, dass**
die Reaktionskomponenten in einer Matrix bereitgestellt werden, bevorzugt einem Gel, besonders bevorzugt einem Agarosegel und wobei die Reaktionskomponenten bei Zugabe der Reaktionslösung und/oder Wärmezufuhr freigegeben werden.

5. Verfahren nach mindestens einem der vorher genannten Ansprüche,
**dadurch gekennzeichnet, dass**
der Amplifikationsschritt eine digitale PCR und/oder eine digitale Festphasen-PCR ist.

6. Verfahren nach mindestens einem der vorher genannten Ansprüche,
**dadurch gekennzeichnet, dass**
die Reaktionslösung Nukleinsäuren, Proteine, Naturstoffe, interkalierenden Farbstoffe, Enzyme, Zusatzstoffe, Viren, Prokaryoten, Eukaryoten, Fragmente aus synthetischen Molekülen und/oder Biomolekülen umfasst.

7. Verfahren nach mindestens einem der vorher genannten Ansprüche,
**dadurch gekennzeichnet, dass**
die Grundfläche 2 bis 10^10, bevorzugt 10^2 bis 10^8, besonders bevorzugt 10^4 bis 10^6 Kavitäten aufweist und/oder die Kavitäten ein Volumen von 1 fL bis 1 mL besitzen.

8. Verfahren nach mindestens einem der vorher genannten Ansprüche,
**dadurch gekennzeichnet, dass**
die Flüssigkeits-Barriere eine chemische und/oder physikalische Barriere ist und/oder die Grundfläche zusätzlich ein Reservoir zur Aufnahme von überschüssiger Reaktionslösung aufweist.

9. Verfahren nach mindestens einem der vorher genannten Ansprüche,
**dadurch gekennzeichnet, dass**
die Grundfläche aus halbleitenden amorphen, kristallinen, quasi-kristallinen und/oder faserartigen Materialien besteht und/oder die Abdeckvorrichtung aus halbleitenden amorphen, kristallinen und/oder faserartigen Materialien, bevorzugt Glas oder Polymer besteht.

10. Verfahren nach mindestens einem der vorher genannten Ansprüche,
**dadurch gekennzeichnet, dass**
die Produktmoleküle anschließend analysiert, sequenziert und/oder derivatisiert werden.

11. Vorrichtung zur Durchführung eines Verfahrens nach mindestens einem der Ansprüche 1 bis 10, wobei
die Vorrichtung eine Grundfläche mit Kavitäten und
eine Abdeckvorrichtung umfassend Spots umfasst, und wobei
die Abdeckvorrichtung bevorzugt ein Mikroarray ist, und wobei
die Abdeckvorrichtung Fängermoleküle umfasst, welche an der Abdeckvorrichtung immobilisiert sind, wobei
die Grundfläche und die Abdeckvorrichtung so zueinander angeordnet sind, dass Reaktionsräume entstehen
**dadurch gekennzeichnet, dass**
die Grundfläche eine Flüssigkeits-Barriere aufweist, welche die Kavitäten umrandet, wobei die Flüssigkeits-Barriere bevorzugt eine chemische und/oder physikalische Barriere ist und/oder
die Grundfläche zusätzlich einen Bereich zur Bereitstellung der Reaktionslösung aufweist und/oder die Grundfläche zusätzlich ein Reservoir zur Aufnahme von überschüssiger Reaktionslösung aufweist
und wobei ein Spot der Abdeckvorrichtung mehrere Kavitäten der Grundfläche abdeckt.

## Claims

1. Method for detection of at least one target molecule and/or product molecule, comprising the following steps:
a) making available a base surface with open cavities and a covering device, wherein
(i) reaction components are present on the base surface and/or the covering device, in defined regions, wherein the defined regions lie on the base surface within the area of the cavities and there are at least two defined regions with different reaction components, and
(ii) the base surface has a liquid barrier that forms an edge around the cavities of the base surface, and a region for making the reaction solution available, and
(iii) the covering device comprises spots and a spot of the covering device covers multiple cavities of the base surface,
b) adding the reaction solution to the region for making the reaction solution available, wherein the reaction solution contains the target molecules,
c) placing the covering device on the base surface, so that the covering device is arranged within the liquid barrier, wherein the reaction solution is distributed among the cavities by means of placing the covering device on, the reaction solution penetrates into the cavities, and each cavity, together with the covering device, forms a closed reaction space after placing on the covering device,
d) carrying out an amplification reaction, a detection reaction and/or a derivatization reaction, wherein the reaction solution comes into contact with the reaction components and product molecules are formed, and
e) detecting at least one target molecule and/or product molecule, wherein at least two different sections of the target molecule and/or product molecule are detected in separate, closed reaction spaces and/or at least two different target molecules and/or product molecules are detected in separate, closed reactions spaces.

2. Method according to claim 1,
**characterized in that**
the covering device is a microarray and the defined regions are spots of the microarray and/or the defined regions lie in the cavities of the base surface.

3. Method according to claim 1 or 2,
**characterized in that**
the reaction components contain primers and/or probes and/or the reaction components or parts of them are immobilized on the base surface and/or the covering surface.

4. Method according to at least one of the preceding claims,
**characterized in that**
the reaction components are made available in a matrix, preferably a gel, particularly preferably an agarose gel, and wherein the reaction components are released when the reaction solution is added and/or heat is supplied.

5. Method according to at least one of the preceding claims,
**characterized in that**
the amplification step is digital PCR and/or digital solid-phase PCR.

6. Method according to at least one of the preceding claims,
**characterized in that**
the reaction solution comprises nucleic acids, proteins, natural substances, intercalating pigments, enzymes, additives, viruses, prokaryotes, eukaryotes, fragments of synthetic molecules and/or biomolecules.

7. Method according to at least one of the preceding claims,
**characterized in that**
the base surface has 2 to 10^10, preferably 10^2 to 10^8, particularly preferably 10^4 to 10^6 cavities and/or that the cavities possess a volume of 1 fL to 1 mL.

8. Method according to at least one of the preceding claims,
**characterized in that**
the liquid barrier is a chemical and/or physical barrier and/or
the base surface additionally has a reservoir for accommodating excess reaction solution.

9. Method according to at least one of the preceding claims,
**characterized in that**
the base surface consists of semiconductive amorphous, crystalline, quasi-crystalline and/or fiber-type materials and/or the covering device consists of semiconductive amorphous, crystalline, quasi-crystalline and/or fiber-type materials, preferably glass or polymer.

10. Method according to at least one of the preceding claims,
**characterized in that**
the product molecules are subsequently analyzed, sequenced and/or derivatized.

11. Apparatus for carrying out a method according to at least one of claims 1 to 10,
wherein
the apparatus comprises a base surface with cavities and
a covering device, comprising spots and wherein
the covering device is preferably a microarray, and wherein
the covering device comprises capture molecules that are immobilized on the covering device, wherein
the base surface and the covering device are arranged, relative to one another, in such a manner that reaction spaces are formed,
**characterized in that**
the base surface has a liquid barrier that forms an edge around the cavities, wherein the liquid barrier is preferably a chemical and/or physical barrier and/or
the base surface additionally has a region for making the reaction solution available and/or the base surface additionally has a reservoir for accommodation of excess reaction solution
and wherein a spot of the covering device covers multiple cavities of the base surface.

## Revendications

1. Procédé de détection d'au moins une molécule cible et/ou d'une molécule de produit, comprenant les étapes suivantes :
a) la mise à disposition d'une surface de base avec des cavités ouvertes et un dispositif de couverture, dans lequel
(i) des composants réactionnels sont présents sur la surface de base et/ou le dispositif de couverture, dans des régions définies, les régions définies se trouvant sur la surface de base dans la zone des cavités et il y a au moins deux régions définies avec des composants réactionnels différents, et
(ii) la surface de base a une barrière aux liquides qui forme un bord autour des cavités de la surface de base, et une région pour mettre à disposition la solution réactionnelle, et
(iii) le dispositif de couverture comprend des points et un point du dispositif de couverture recouvre de multiples cavités de la surface de base,
b) l'addition de la solution réactionnelle à la région permettant de mettre à disposition la solution réactionnelle, la solution réactionnelle contenant les molécules cibles,
c) le placement du dispositif de couverture sur la surface de base, de sorte que le dispositif de couverture est agencé à l'intérieur de la barrière aux liquides, la solution réactionnelle étant distribuée entre les cavités par la mise en place du dispositif de couverture, la solution réactionnelle pénétrant dans les cavités, et chaque cavité, avec le dispositif de couverture, formant un espace réactionnel clos après la mise en place du dispositif de couverture,
d) la réalisation d'une réaction d'amplification, d'une réaction de détection et/ou d'une réaction de dérivation, la solution réactionnelle entrant en contact avec les composants réactionnels et des molécules de produit sont formées, et
e) la détection d'au moins une molécule cible et/ou d'une molécule de produit, au moins deux sections différentes de la molécule cible et/ou de la molécule de produit étant détectées dans des espaces réactionnels clos séparés et/ou au moins deux molécules cibles et/ou molécules de produit différentes étant détectées dans des espaces réactionnels clos séparés.

2. Procédé selon la revendication 1,
**caractérisé en ce que**
le dispositif de couverture est un microréseau et les régions définies sont des points du microréseau et/ou les régions définies se trouvent dans les cavités de la surface de base.

3. Procédé selon la revendication 1 ou 2,
**caractérisé en ce que**
les composants réactionnels contiennent des amorces et/ou des sondes et/ou les composants réactionnels ou des parties de ceux-ci sont immobilisés sur la surface de base et/ou la surface de couverture.

4. Procédé selon au moins l'une des revendications précédentes,
**caractérisé en ce que**
les composants réactionnels sont mis à disposition dans une matrice, de préférence un gel, de manière particulièrement préférée un gel d'agarose, et les composants réactionnels étant libérés lorsque la solution réactionnelle est ajoutée et/ou de la chaleur est fournie.

5. Procédé selon au moins l'une des revendications précédentes,
**caractérisé en ce que**
l'étape d'amplification est une PCR numérique et/ou une PCR numérique en phase solide.

6. Procédé selon au moins l'une des revendications précédentes,
**caractérisé en ce que**
la solution réactionnelle comprend des acides nucléiques, des protéines, des substances naturelles, des pigments intercalants, des enzymes, des additifs, des virus, des procaryotes, des eucaryotes, des fragments de molécules synthétiques et/ou des biomolécules.

7. Procédé selon au moins l'une des revendications précédentes,
**caractérisé en ce que**
la surface de base a de 2 à 10^10, de préférence de 10^2 à 10^8, de manière particulièrement préférée de 10^4 à 10^6 cavités et/ou **en ce que** les cavités possèdent un volume de 1 fL à 1 mL.

8. Procédé selon au moins l'une des revendications précédentes,
**caractérisé en ce que**
la barrière aux liquides est une barrière chimique et/ou physique et/ou
la surface de base comporte en outre un réservoir pour recevoir la solution réactionnelle en excès.

9. Procédé selon au moins l'une des revendications précédentes,
**caractérisé en ce que**
la surface de base est constituée de matériaux semi-conducteurs amorphes, cristallins, quasi-cristallins et/ou fibreux et/ou le dispositif de couverture est constitué de matériaux semi-conducteurs amorphes, cristallins, quasi-cristallins et/ou fibreux, de préférence de verre ou d'un polymère.

10. Procédé selon au moins l'une des revendications précédentes,
**caractérisé en ce que**
les molécules de produit sont ensuite analysées, séquencées et/ou dérivées.

11. Appareil pour la mise en oeuvre d'un procédé selon au moins l'une des revendications 1 à 10, dans lequel
l'appareil comprend une surface de base avec des cavités et
un dispositif de couverture, comprenant des points et dans lequel
le dispositif de couverture est de préférence un microréseau, et dans lequel
le dispositif de couverture comprend des molécules de capture qui sont immobilisées sur le dispositif de couverture, dans lequel
la surface de base et le dispositif de couverture sont agencés l'un par rapport à l'autre de manière à former des espaces réactionnels,
**caractérisé en ce que**
la surface de base présente une barrière aux liquides qui forme un bord autour des cavités, la barrière aux liquides étant de préférence une barrière chimique et/ou physique et/ou
la surface de base comporte en outre une région pour la mise à disposition de la solution réactionnelle et/ou la surface de base comporte en outre un réservoir pour recevoir une solution réactionnelle en excès et dans lequel un point du dispositif de couverture recouvre de multiples cavités de la surface de base.
